(19) Europäisches Patentamt
European Patent Office
Office européen des brevets

(11) EP 1 834 637 A1

(12) **EUROPEAN PATENT APPLICATION**
published in accordance with Art. 158(3) EPC

(43) Date of publication:
**19.09.2007 Bulletin 2007/38**

(21) Application number: **05770604.6**

(22) Date of filing: **05.08.2005**

(51) Int Cl.:
***A61K 31/01*** *(2006.01)*

(86) International application number:
**PCT/JP2005/014798**

(87) International publication number:
**WO 2006/061925 (15.06.2006 Gazette 2006/24)**

(84) Designated Contracting States:
**GB**

(30) Priority: **07.12.2004 JP 2004354271**

(71) Applicant: **Vitamin C60 Bioresearch Corporation**
**Tokyo 100-0005 (JP)**

(72) Inventors:
- **MIWA, Nobuhiko,**
**Hiroshima Prefectural University**
**Shobara-shi,**
**Hiroshima 727-0023 (JP)**
- **ITO, Shinobu,**
**Higashi-biru 7F, 10-1**
**Musashino-shi,**
**Tokyo 180-0003 (JP)**
- **MATSUBAYASHI, Kenji,**
**Mitsubishi Corporation**
**Tokyo 100-0005 (JP)**

(74) Representative: **Matthews, Derek Peter**
**Frank B. Dehn & Co.**
**St Bride's House**
**10 Salisbury Square**
**London EC4Y 8JD (GB)**

# (54) PREVENTIVE/THERAPEUTIC COMPOSITION FOR FREE RADICAL DISEASE

(57) A preventive/therapeutic composition for free radical diseases, **characterized by** containing as an active ingredient at least one kind of a fullerene, a fullerene derivative, and a composite comprising a fullerene or fullerene derivative and an organic compound with which the fullerene or derivative has been modified or clathrated. The composition is reduced in side effects, has the high ability to eliminate free radicals in the body, and further has excellent preparation stability.

**Fig. 1**

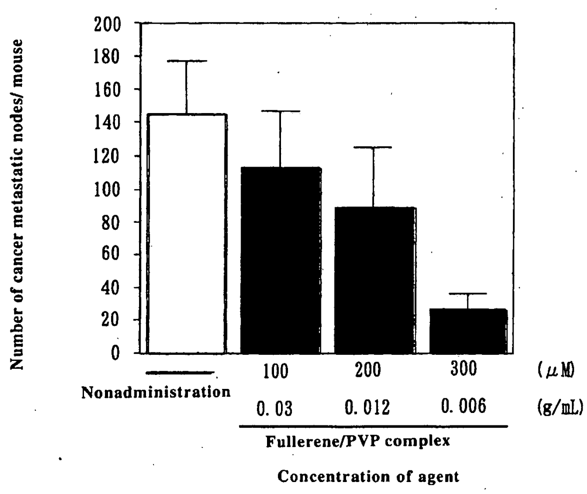


EP 1 834 637 A1

## Description

Technical Field

The present invention relates to a preventive/ therapeutic composition for a free radical disease, which exhibits a preventive effect or a therapeutic effect on various diseases associated with free radical, and insures the stability of a pharmaceutical preparation and has high safety with less side effects in vivo.

Background Art

In recent years, carbon nanostructures such as fullerenes have attracted attention as a carbon material to bring a new development vision, and drawn increasing attention on application thereof to not only an electronic material or an electrode material, but also to medical service and medical science, or for the purpose of promotion of health and the like.

A fullerene molecule is composed of carbon atoms and includes 32 to 100 or more of carbon atoms in each spherical body. For a more detailed description regarding fullerenes, reference may be made to the following articles: (i) R. E. Smalley, "Supersonic Carbon Cluster Beams in Atomic and Molecular Clusters", edited by E. R. Berstein, Physical and Theoretical Chemistry, vol. 68, Elsevier Science, (New York), pp. 1-68, 1990; (ii) R. F. Curl et al., "Fullerenes" Scientific American, pp. 32-41, October, 1991; (iii) F. Diederich et al., "The Higher Fullerenes: Isolation and Characterization", Science; vol. 252, pp. 548-551, April, 1991; and (iv) R. E. Smalley, "Great Balls of Carbon: The Story of Buckminsterfullerene", The Sciences, vol. 31, No. 2, pp. 22-28, March to April, 1991.

Among such fullerenes, the most typical fullerene is a C60 fullerene, and its structure recalls a football. Other fullerenes, especially C70 as well as C84 exist as higher fullerenes. The fullerenes are now commonly known as "buckyballs". Incidentally, the molecules of C60 fullerenes are generally contaminated with a small amount of a C70 fullerene and a C84 fullerene as impurities. The preparation method of fullerenes together with their solubility, crystallinity and color characteristics have been described in many publications, and in particular in the publications written by W. Kraetschmer et al., "Nature, vol. 347, pp. 354-358, 1990 and Chemical and Engineering News, pp. 22-25, October, 1990".

As the interest in fullerenes increases, the application of fullerenes to a pharmaceutical product or a cosmetic product have been investigated, and a specific proposal has been made. For example, blending a fullerene or a fullerene mixture in a cosmetic product (Patent document 1), preparation of a sun care cosmetic composition by utilizing the UV absorbing effect of a fullerene (Patent document 2), a method of optically inactivating viruses with the use of a fullerene as a photosensitizer (Patent document 3), and application of a fullerene as an antioxidant to a skin preparation for external use and the like (Patent document 4) have been proposed so far by the present inventors.

On the other hand, the incidence rate of adult diseases has increased because of the progress of aging of society, and particularly, various diseases accompanying aging are growing problems. Among the diseases associated with adult diseases, cardiac arrhythmia, arteriosclerosis, an ischemic heart disease, heart failure, myocardial infarction, liver damage, ischemic liver damage, digestive system damage, vascular damage, pancreatic damage, gallbladder damage, organ transplantation damage, diabetes, hypertension, organ failure and the like are typical adult diseases, therefore, a lot of pharmaceutical products for preventing or treating such diseases have been developed.

Further, as other diseases, there are various carcinomas such as lung cancer, stomach cancer and large bowel cancer, symptoms due to various infectious diseases such as hepatitis and acquired immunodeficiency syndrome (AIDS) caused by HIV and the like. It has been pointed out that the fundamental cause of these diseases or one of the causal factors thereof is a disease caused by free radical in vivo (in the present invention, the disease is referred to as a free radical disease) (Non-patent documents 1 and 2). In fact, for the purpose of treating or preventing such a free radical disease, the development of an agent for preventing/treating, and treating a free radical disease has been advanced for a relatively long time, and a large number of pharmaceutical ingredients as described below have been already proposed.

Examples thereof include an SOD-associated substance (Non-patent document 3), a ubiquinone preparation (Patent document 5), a benzoazepine derivative (Patent document 6), a pyrrolidinone derivative (Patent document 7), a substituted vinyl derivative (Patent document 8), an oxamide derivative (Patent document 9), a furancarboxamide derivative (Patent document 10), a phthalimide derivative (Patent document 11), docosahexaenoic acid (Patent document 12), a linolenic acid derivative (Patent document 13), a quinoline oxide derivative (Patent document 14), a heteroaromatic amine derivative (Patent document 15), a carbostyril derivative (Patent document 16), an isoindol derivative (Patent document 17), a single cell producing product (Patent document 18), an imidazolthion carboxamide derivative (Patent document 19), a dibenzooxepine derivative (Patent document 20), a propylamine derivative (Patent document 21), a phenylphthalazine derivative (Patent document 22), a tannic acid derivative (Patent document 23), a glycyrrhetinic acid derivative (Patent document 24), a cancer metastasis inhibitor (for example, Patent documents 25 to 31) and the like.

However, although a part of these compounds has an action of scavenging free radical, it has problems in

terms of a formulation process and stability in vivo. In particular, it has a problem that an active ingredient cannot be delivered at a high concentration to a target organ because it scavenges free radical and loses its activity in other organs before reaching an affected area. Further, there has also been a problem that when the above-mentioned pharmaceutical ingredient is administered in vivo, side effects such as headache, nausea, vomiting, anorexia, sickness, skin rash or twitch are developed.

On the other hand, as a substance having a high activity of scavenging free radical with less side effects, a single substance such as L-ascorbic acid or tocopherol or a derivative thereof is known, and has been used for the prevention or treatment of a free radical disease. However, such an antioxidant substance is more likely to be oxidized and unstable, and is susceptible to oxidative degradation while or even after it is formulated into a preparation, therefore it is difficult to keep the quality thereof as a pharmaceutical preparation for a long period of time. Further, even in an injectable agent or the like containing such an antioxidant substance, a heat sterilization treatment such as an autoclave treatment cannot be carried out, therefore, there has been a problem that it cannot be incorporated in vivo in a stable state by a method such as intravenous injection. Further, there has been a problem that even if such a conventional antioxidant is administered orally or intravenously, its activity is liable to be lost in vivo, and moreover, it is easily metabolized and discharged from the body promptly, therefore, an effect on scavenging free radical cannot be sufficiently exhibited.

Accordingly, based on the knowledge obtained so far by the present inventors that a fullerene is applied to a skin preparation for external use or the like as an antioxidant, an object of the present invention is to provide a new preventive/therapeutic composition for a free radical disease, which solves the problems of the conventional drugs and agents related to the prevention or treatment of a free radical disease as described above, has less side effects, exhibits a high activity of scavenging free radical in vivo, and is also excellent in stability as a pharmaceutical.

Patent document 1: JP-A-6-192039
Patent document 2: JP-A-9-278625
Patent document 3: JP-A-9-322767
Patent document 4: JP-A-2004-250690
Patent document 5: JP-A-57-42616
Patent document 6: JP-A-57-193462
Patent document 7: JP-A-59-27823
Patent document 8: JP-A-60-28963
Patent document 9: JP-A-59-163353
Patent document 10: JP-A-59-190991
Patent document 11: JP-A-61-37766
Patent document 12: JP-A-4-273817
Patent document 13: JP-A-61-44853
Patent document 14: JP-A-61-65869
Patent document 15: JP-A-61-282377
Patent document 16: JP-A-63-301821
Patent document 17: JP-A-63-150276
Patent document 18: JP-A-1-143868
Patent document 19: JP-A-1-146820
Patent document 20: JP-A-3-176487
Patent document 21: JP-A-3-236378
Patent document 22: JP-A-4-211666
Patent document 23: JP-A-1-25726
Patent document 24: JP-A-4-330088
Patent document 25: JP-A-54-4401
Patent document 26: JP-A-2-308799
Patent document 27: JP-A-4-312531
Patent document 28: JP-A-6-72871
Patent document 29: JP-A-6-107693
Patent document 30: JP-A-8-291075
Patent document 31: JP-A-10-330261
Non-patent document 1: "Kassei Sanso to Byotai (Free Radical and Pathologic Conditions)" edited by Inoue, Japan Scientific Societies Press, 1992
Non-patent document 2: "Kasanka Busshitsu (Peroxides)" written by Niki, et al., Japan Scientific Societies Press, 1994
Non-patent document 3: Inoue, et al., Biochemistry, 28, 6619-6624, 1989

Disclosure of Invention

Recently, the present inventor confirmed that when a vascular endothelial cell line was cultured in vitro as an adult disease model and free radical is generated by subjecting the cell to a temporary low oxygen condition, and then, a fullerene was allowed to act thereon, the generation of free radical in the cell was suppressed more compared with the case of vitamin C which is conventionally used. Further, it was found that a fullerene is much more excellent in stability, has low toxicity and can exhibit an effect sufficiently in vivo, thus the present invention has been completed.

That is, the preventive/therapeutic composition for a free radical disease of the present invention is characterized by the following aspects as the one that achieves the above object.

1: A preventive/therapeutic composition for a free radical disease, characterized by comprising as an active ingredient, at least one kind of a fullerene, a fullerene derivative, and a complex of a fullerene or a fullerene derivative modified with or clathrated in an organic compound.

2: The preventive/therapeutic composition for a free radical disease according to the above 1, wherein the fullerene derivative is a fullerene linked to at least one kind of an oxygen-containing group, a nitrogen-containing group and a hydrocarbon group which may have a substituent.

3: The preventive/therapeutic composition for a free radical disease according to the above 1, wherein the fullerene is at least one kind selected from a fullerene polymer, a carbon nanotube, a derivative thereof, and a salt thereof.

4: The preventive/therapeutic composition for a free radical disease according to the above 1, wherein the fullerene is at least one kind selected from a cleaved form of a fullerene, a split form of a fullerene, a derivative thereof and a salt thereof.

5: The preventive/therapeutic composition for a free radical disease according to the above 3 or 4, wherein the salt of a fullerene is at least one kind of salts of sodium, potassium, magnesium, calcium and aluminum.

6: The preventive/therapeutic composition for a free radical disease according to any one of the above 1 to 5, wherein the fullerene is a complex of at least one kind of a fullerene and a fullerene derivative with at least one kind of an organic oligomer, an organic polymer, cyclodextrin, crown ether and an analogous compound thereof.

7: The preventive/therapeutic composition for a free radical disease according to the above 6, which is a complex of at least one kind of a fullerene and a fullerene derivative with polyvinylpyrrolidone (PVP).

8: The preventive/therapeutic composition for a free radical disease according to any one of the above 1 to 5, wherein the fullerene is represented by the following formula:

$$R1m\text{-}F\text{-}R2m$$

(wherein F denotes a fullerene represented by Cn (C denotes a carbon atom and n denotes an integer of 32 or more), a carbon nanotube, a polymer thereof, a cleaved form thereof, a split form thereof or a mixture thereof; R1m denotes m-number of substituents linked to Cn, the substituents are independently the same or different and represent a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; R2m denotes m-number of atoms linked to Cn, the atoms are independently the same or different or one atom is linked to 2 or more different carbons; and m denotes 0 or an integer of 1 or more).

9: The preventive/therapeutic composition for a free radical disease according to the above 4 or 8, wherein the cleaved form or split form of a fullerene is a fullerene represented by any of the following formulae or a single substance or a complex of a molecule having the structure therein:

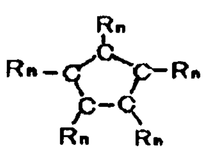

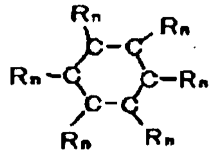

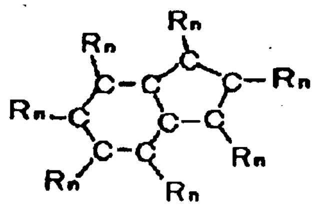

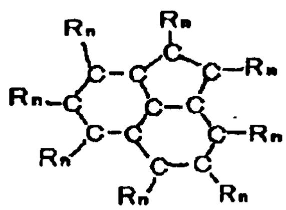

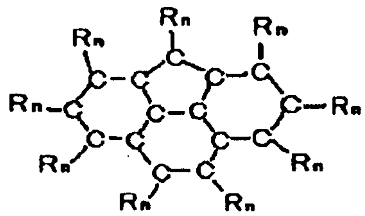

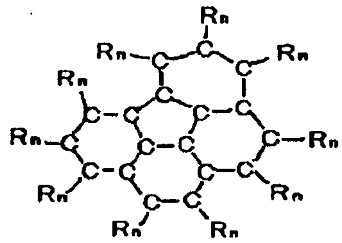

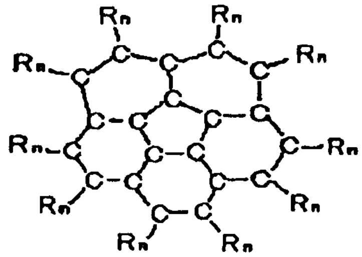

(wherein C denotes a partial structure of a fullerene represented by a carbon atom and 2 or more carbon atoms may be linked via a covalent bond (in the case where a carbon which is not covalently linked is present, even if there is no denotation by Rn in the formula, the carbon is linked to Rn); Rn denotes n-number of substituents linked to C, the substituents are independently the same or different and represent a hydrogen atom, a carbon atom, an oxygen atom, a nitrogen atom, a phosphorous atom, a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; and n denotes 0 or an integer of 1 or more).

10: The preventive/therapeutic composition for a free radical disease according to any one of the above 1 to 9, further comprising at least one kind of antitumor agent.

11: The preventive/therapeutic composition for a free radical disease according to the above 10, wherein the antitumor agent is at least one kind of nitromin (R), cyclophosphamide, merphalan, thiotepa, carboquone, Protecton (R), busulfan, nimustine hydrochloride, mitobronitol, ifosfamide, mercaptopurine, thioinosine, cytarabine, dacarbazine, fluorouracil, tegaful, ancitabine hydrochloride, methotrexate, carmofur, UFT (R), enocitabine, vinblastine sulfate, vincristine sulfate, vindesine sulfate, actinomycin (D), mitomycin C, chromomycin A3, bleomycin hydrochloride, bleomycin sulfate, daunorubicin hydrochloride, doxorubicin hydrochloride, neocarzinostatin, peplomycin sulfate, aclarubicin hydrochloride, mepitiostane, epitiostanol, tamoxifen citrate, Honvan, Picibanil (R), krestin, lentinan, L-asparaginase, aceglatone, procarbazine hydrochloride, floxuridine, MDS KOWA 3000 (R), cisplatin, Estracyt (R), Sizofiran, protamine, an angiostatic steroid in the presence of heparin, an antitumor polysaccharide, a laminin peptide, an antitumor polypeptide containing an Arg-Gly-Asp (RGD) sequence and an antitumor platelet factor.

12: The preventive/therapeutic composition for a free radical disease according to any one of the above 1 to 11, wherein the free radical disease is myocardial infarction, an ischemic heart disease, heart failure, angina pectoris, cardiac arrhythmia, arteriosclerosis, disturbance of lipid metabolism in the liver, hyperlipemia, essential hypertension, hypertension, arteriosclerosis, coronary arteriosclerosis, thrombosis, arteriosclerosis obliterans, vascular disorder, peripheral vascular disorder, cholestasis, hypercholesterolemia, pancreatic damage, organ failure, acute or chronic hepatitis, gastric ulcer, duodenal ulcer, colitis ulcerosa, digestive system damage, cholecystopathy, diabetes, arthritis therapeutic agent, rheumatoid, liver failure, liver damage, ischemic liver damage, disturbance of lipid metabolism in the liver, gallbladder damage, organ transplantation damage, diabetes, toxicosis, organ transplantation damage, cancer, ischemic reperfusion damage, tissue aging, skin pigmentation, skin wrinkle, alveolar pyorrhea, skin seborrhea, skin tanning, skin acne, burn injury, obesity, hair loss, mental disorder, dementia, Parkinson’s disease or an infectious disease.

13: The preventive/therapeutic composition for a free radical disease according to the above 12, wherein the cancer is a tumor, a benign tumor or a malignant tumor.

14: The preventive/therapeutic composition for a free radical disease according to the above 12, wherein the cancer is malignant melanoma, malignant lymphoma, gastrointestinal cancer, lung cancer, esophageal cancer, stomach cancer, large bowel cancer, rectal cancer, colon cancer, ureteral tumor, gallbladder cancer, bile duct cancer, biliary tract cancer, breast cancer, liver cancer, pancreatic cancer, testicle tumor, maxillary cancer, tongue cancer, lip cancer, oral cavity cancer, pharyngeal cancer, larynx cancer, ovary cancer, uterine cancer, prostate cancer, thyroid cancer, brain tumor, Kaposi’s sarcoma, hemangioma, leukemia, polycythemia vera, neuroblastoma, retinoblastoma, myeloma, bladder tumor, sarcoma, osteogenic sarcoma or myosarcoma.

15: The preventive/therapeutic composition for a free radical disease according to the above 12, wherein the cancer is skin cancer, basal cell cancer, skin appendage carcinoma, skin metastasis cancer or skin melanoma.

16: The preventive/therapeutic composition for a free radical disease according to the above 12, wherein the ischemic reperfusion damage is an ischemic heart disease, ischemic reperfusion myocardial damage, ischemic liver damage, ischemic reperfusion liver damage, ischemic reperfusion renal damage, ischemic reperfusion pancreatic damage, ischemic reperfusion gallbladder damage, ischemic reperfusion cardiovascular damage, ischemic reperfusion gastrointestinal damage, ischemic reperfusion muscle damage, ischemic reperfusion vascular damage, ischemic reperfusion eye damage or ischemic reperfusion skin damage.

17: The preventive/therapeutic composition for a free radical disease according to the above 12, wherein the infectious disease is a bacterial infectious disease, a viral infectious disease, a fungal infectious disease or a parasitic infectious disease.

18: The preventive/therapeutic composition for a free radical disease according to the above 17, wherein the viral infectious disease is hepatitis, acquired immunodeficiency syndrome (AIDS), common cold or influenza.

Brief Description of Drawings

Fig. 1 is a view showing an effect of PVP/fullerene on suppressing skin cancer metastasis in Test example 5.
Fig. 2 is a view showing an effect of PVP/fullerene on suppressing cancer cell metastasis in Test example 6.

Best Mode for Carrying Out the Invention

The invention of this application has characteristics as described above, and, hereinafter an embodiment thereof will be described.

In the preventive/therapeutic composition for a free radical disease of the present invention, an active ingredient or an effective ingredient is a fullerene as described above, and is composed of at least one kind of a fullerene, a fullerene-containing oxygen derivative, the fullerene and the fullerene-containing oxygen derivative modified with or clathrated in an organic compound, and a salt thereof.

The fullerene among these fullerenes is represented by Cn (n denotes an integer of 60 or more) and may be any kind such as C60, C70 or a mixture thereof, and further, a conventionally known one having a carbon skeletal structure in the form of a sphere, a tube or the like, including a carbon tube fullerene. For example, the fullerene of the invention of this application may be the one in which plural fullerenes are linked through an alkylene chain such as a methylene chain, the one in which an alkylene chain is linked to a carbon atom at a different location in the fullerene skeleton, or the like. As the derivative of a C60 fullerene, 1 to 40 modifying groups may be linked to one fullerene molecule, and for example, as the derivative of a C70 fullerene, 1 to 50 modifying groups may be linked to one fullerene molecule. Such modifying groups may be independently a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde. The fullerene. of the invention may be such a fullerene-modified compound, a salt thereof or at least one kind selected therefrom. Further, the fullerene of the invention of this application may be a C60 fullerene, a C70 fullerene or a nanotube fullerene, or it may be a mixture of one or more kinds selected therefrom. In addition, it may be a fullerene including remaining carbon black (soot including a fullerene), which is an unpurified product of the fullerene, and may be a fullerene in which the concentration of carbon black is 0 to 98% by weight.

Further, in the fullerene of the invention of this application, a fullerene derivative having in the carbon skeletal structure as described above, any of various substituents including a hydrocarbon group which may have a substituent, an oxygen-containing group such as an oxygen-crosslinked group, a hydroxyl group, an acyl group, an ether group or a carboxyl group, a nitrogen-containing group such as an amino group or a cyano group and the like is also included.

With regard to the fullerene-containing oxygen derivative, the one in which an oxygen atom is linked directly to a carbon atom of the fullerene skeleton or through a carbon chain such as an alkylene chain is considered. For example, a hydroxylated fullerene or the like in which hydroxyl groups are directly linked at a hydroxylation rate of about 50/mol of fullerene or less can be exemplified.

As an example of the fullerene that can be used in the present invention, a fullerene represented by the following chemical formula can be used.

**R1m-F-R2m**

(In the formula, F denotes a fullerene represented by Cn (C denotes a carbon atom and n denotes an integer of 32 or more), a carbon nanotube, a polymer thereof, a cleaved form thereof, a split form thereof or a mixture thereof; R1m denotes m-number of substituents linked to Cn, the substituents are independently the same or different and represent a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; R2m denotes m-number of atoms linked to Cn, the atoms are independently the same or different or one atom is linked to 2 or more different carbons; and m denotes 0 or an integer of 1 or more).

Further, the fullerene that can be used in the present invention may be a single substance of at least one kind selected from a fullerene polymer, a carbon nanotube, a derivative thereof and a salt thereof, or a mixture thereof, or an organic compound with or in which these are modified or clathrated.

Examples of the fullerene polymer include those represented by the following formulae. That is, there are a dimer of $C_{60}$ ($C_{120}$), a trimer thereof (C180, see the following drawing), the synthesis of which was achieved by the group of Komatsu et al. in Kyoto University by placing potassium cyanide and an iron ball for grinding in a capsule made of iron steel and providing a high speed vibration of 3500 cycles per minute, C62 synthesized by Rubin et al., a nitrogen linked fullerene $(C_{59}N)_2$ synthesized by Wudl et al., and the like.

Further, examples of the derivative of a fullerene that can be used in the present invention include $C_{60}F$, $C_{60}F_{18}$ obtained by reacting fluorine with a fullerene and the like.

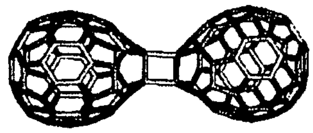

$C_{120}$

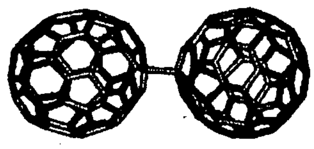

$(C_{59}N)_2$

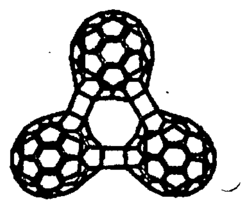

$C_{180}$

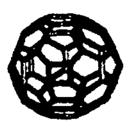

$C_{62}$

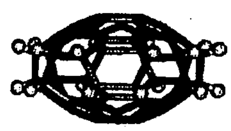

$C_{60}F_{20}$

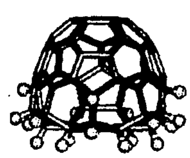

$C_{60}F_{18}$

Further, in the present invention, for example, a metal-encapsulated fullerene (M in the formula denotes a metal atom) as represented by the following formula synthesized by mixing a metal in graphite powder and applying a laser beam to the mixture so as to effect evaporation can also be used. Specific examples of the metal-encapsulated fullerene that can be used in the present invention include scandium-, lanthanum-, cesium-, titanium-encapsulated fullerenes and the like, however, it is not limited to these.

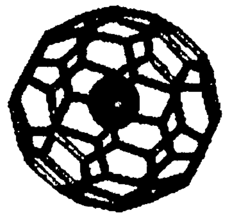

**Metal-encapsulated fullerene ($M@C_{60}$)**

As the fullerene that can be used in the present invention, for example, an atom-encapsulated fullerene (a microsphere in the formula denotes an atom) as represented by the following formula can also be used.

Specific examples thereof include $(Sc_3N)@C_{80}$ and $(Sc_2C_2)@C_{84}$ encapsulating $Sc_3N$ and $Sc_2C_2$, respectively and the like.

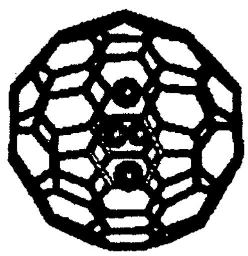

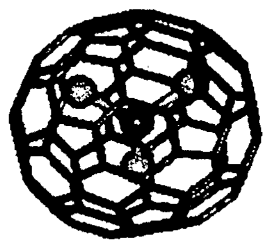

**$(Sc_2C_2)@C_{84}$ and $(Sc_3N)@C_{80}$**

As the fullerene that can be used in the present invention, a single substance of at least one kind selected from a cleaved form of a fullerene, a split form of a fullerene, a derivative thereof and a salt thereof or a mixture thereof can be exemplified.

For example, examples of the cleaved fullerene that can be used in the present invention include an open-cage fullerene produced by Rubin et al. as represented by the following formula, a fullerene with holes synthesized by Komatsu et al., and the like.

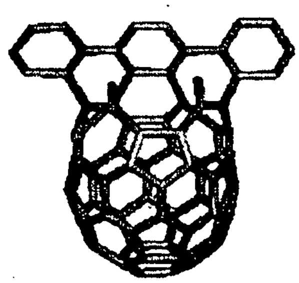

**Open-cage fullerene**

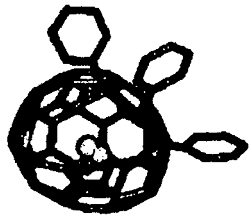

**Fullerene with holes**

In the fullerene that can be used in the present invention, a complex of a fullerene with an organic compound for modifying or clathrating the fullerene or forming a conjugate thereof. As such an organic compound, for example, one or more kinds of an organic oligomer, an organic polymer, cyclodextrin (CD) or crown ether capable of forming a clathrate compound or a clathrate complex and an analogous compound thereof are preferably exemplified.

As the organic oligomer or the organic polymer, for example, a carboxylic acid ester, an alcohol, a saccharide, a polysaccharide, a polyhydric alcohol, a polyalkylene glycol such as polyethylene glycol, butylene glycol, polypropylene glycol or polyvinyl alcohol, or a polymer of a polyhydric alcohol, a nonionic water-soluble polymer including dextran, pullulan, starch, and a starch derivative such as hydroxyethyl starch or hydroxypropyl starch, alginic acid, hyaluronic acid, chitosan, a chitin derivative, an anionic or a cationic derivative of a polymer thereof, a high molecular weight glycerin and a fatty acid thereof, an oil, propylene carbonate, lauryl alcohol, ethoxylated caster oil, a polysorbate, an ester or an ether thereof, a polymer thereof, a polyester polymer thereof, a pyrrolidone polymer such as polyvinylpyrrolidone, an ester or an ether of an unsaturated alcohol polymer, a block copolymer of polyoxyethylene and polyoxypropylene or the like may be used, and the one in which any of these is linked to a fullerene or a derivative thereof is preferred, and it may be a mixture of one or more of these. In particular, various types of polyalkylene glycols such as polyethylene glycol (PEG) and polyvinylpyrrolidone (PVP) are preferably exemplified. In the case of a polymer of PEG, PVP or the like, with regard to the average molecular weight thereof, about 2,000 to 100,000 is generally preferred, and with regard to the ratio thereof to the fullerene or the fullerene-containing oxygen derivative, about 10/1 or less in terms of a molar ratio is considered.

The salt of the fullerene of the invention of this application may be selected from, for example, a salt of a hydroxylated fullerene or a fullerene ester, a salt of a polyhydroxylated fullerene, a fullerene diester, a fullerene triester, a fullerene polyester and the like, and such a salt may be any as long as a physiologically acceptable salt is formed. Examples of such a salt include an inorganic salt and an organic salt.

Such a salt may be a salt with a metal such as an alkali metal salt (for example, sodium, potassium or the like), or an alkaline earth metal salt (for example, calcium, magnesium or the like), or may be a salt with an organic base such as trimethylamine, triethylamine, pyridine, picoline, N,N-dibenzylethylenediamine, ethanolamine, diethanolamine, trishydroxymethyl aminomethane, or dicyclohexylamine. In view of high safety, economic advantage and the like, as the salt of a hydroxylated fullerene or a fullerene monoester, a metal salt of one or more kinds selected from sodium, potassium, magnesium, calcium and aluminum is preferably used.

Further, in the fullerene that can be used in the present invention, for example, a clathrate compound with cyclodextrin or the like, a porphyrin complex synthesized by Boyd et al. (jaws porphyrin), a double nanoring cyclophane fullerene complex, a fullerene-ferrocene derivative synthesized by Nakamura et al., a fullerene- phenyl derivative $Ph_5C_{60}$, a fullerene-biphenyl derivative as shown in the following formulae and the like are also included.

As the fullerene, a water-soluble fullerene derivative, a fullerene modified with or clathrated in an organic compound, and a salt of a fullerene have high stability, are soluble in water, can be adjusted in terms of their pH, therefore, they have low cytotoxicity, and have high biocompatibility, thus, they are suitable to be used in the invention of this application. For example, a fullerene modified with or clathrated in PEG (polyethylene glycol), PVP (polyvinylpyrrolidone) or CD (cyclodextrin) as a high molecular weight polymer is preferred, and because a monovalent salt has a higher solubility in water than a divalent salt, therefore, it is preferred. In particular, fullerene/PVP, a sodium salt of a hydroxylated fullerene or a fullerene ester, a potassium salt of a hydroxylated fullerene or a fullerene monoester are suitable.

For example, as one of the fullerenes which have drawn attention, there is a water-soluble PVP/fullerene complex. PVP (polyvinylpyrrolidone) is represented by the following formula.

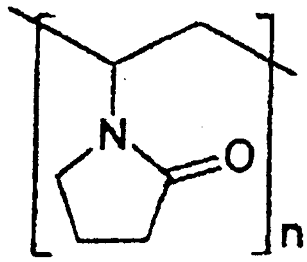

Polyvinylpyrrolidone (PVP)

In the invention of this application, the one with a weight average molecular weight (Mw) of from about 3,000 to 3,000,000, more preferably, the one with a weight average molecular weight (Mw) of from about 6,000 to 1,500,000 is considered as a preferred one. PVP may be the one obtained by synthesis or may be a commercially available one. The fullerene in this case may be a fullerene derivative as described above.

Further, in the fullerene that can be used in the present invention, for example, a clathrate compound with cyclodextrin or the like, a porphyrin complex synthesized by Boyd et al. (jaws porphyrin), a double nanoring cyclophane fullerene complex, a fullerene-ferrocene derivative synthesized by Nakamura et al., a fullerene- phenyl derivative $Ph_5C_{60}$, a fullerene-biphenyl derivative as shown in the following formulae and the like are also included.

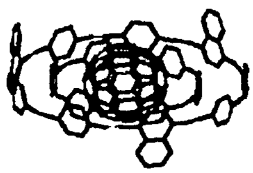

**Double nanoring cyclophane fullerene complex**

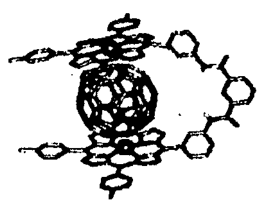

**Jaws porphyrin**

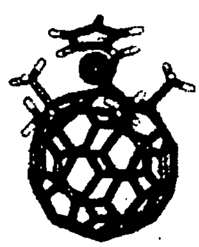

**Fullerene-ferrocene derivative**

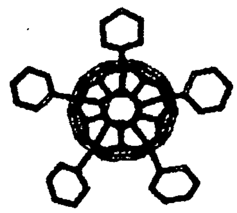

Fullerene-benzene derivative $Ph_5C_{60}$

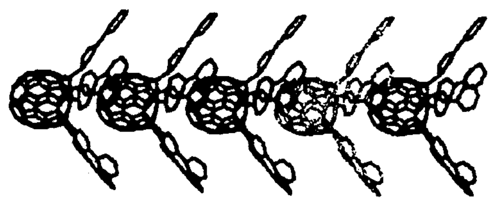

Fullerene-biphenyl derivative complex

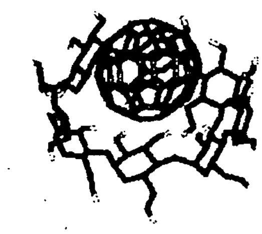

Cyclodextrin clathrate compound of fullerene

Further, as the fullerene derivative that can be used in the present invention, the one in which an oxygen atom is linked directly to a carbon atom of the fullerene skeleton or through a carbon chain such as an alkylene chain is considered. For example, a hydroxylated fullerene in which hydroxyl groups are directly linked at a hydroxylation rate of about 50/mol of fullerene or more and the like can be exemplified.

The cleaved form or the split form of a fullerene that can be used in the present invention may be a cleaved form or a split form of a fullerene represented by any of the following chemical formulae or a single substance or a complex of a molecule having the structure therein.

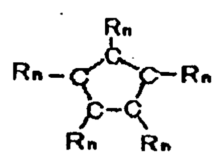

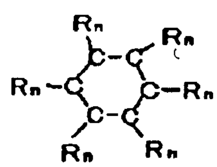

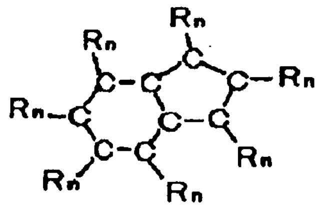

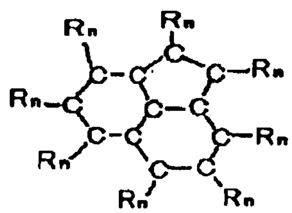

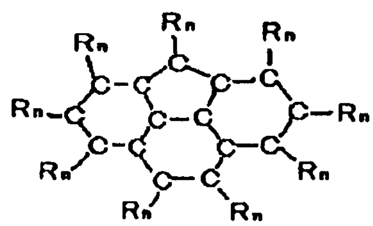

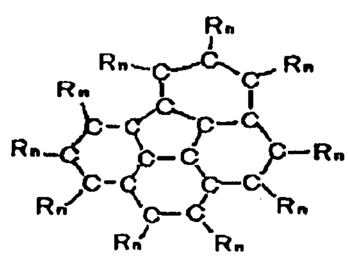

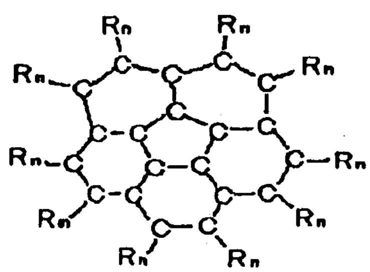

In the formula, C denotes a partial structure of a fullerene represented by a carbon atom and 2 or more carbon atoms may be linked via a covalent bond (in the case where a carbon which is not covalently linked is present, even if there is no denotation by Rn in the formula, the carbon is linked to Rn); Rn denotes n-number of substituents linked to C, the substituents are independently the same or different and represent a hydrogen atom, a carbon atom, an oxygen atom, a nitrogen atom, a phosphorous atom, a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; and n denotes 0 or an integer of 1 or more.

Further, examples of the split fullerene that can be used in the present invention include corannulene ($C_{20}H_{10}$) having a structure of a C60 fullerene fragment as shown by the following formula and the like.

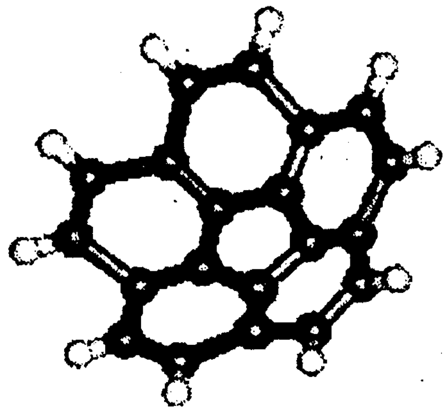

In the invention of this application, it is also effective that together with the fullerene as an active ingredient or an effective ingredient as described above, for example, a long chain carboxylic acid having 10 or more carbon atoms, an ester thereof or a salt thereof is included. Further, an oil, a surfactant, a pigment, a moisturizer, an excipient, a base, a cell activator as described later or the like may also be blended.

In the case of containing moisture in a fullerene- containing composition for external use, the pH thereof, although it varies depending on the pH of a bulk product of a fullerene, a fullerene derivative, a clathrate compound of a fullerene, or a salt thereof, is preferably in the range of from 3 to 10 in general because the fullerene and the derivative thereof can be stably blended.

When the pH of a 0.5% by weight aqueous solution of the bulk product of a fullerene, a fullerene derivative, a clathrate compound of a fullerene or a salt thereof was measured at 20˚C, and the numeric value rounded to the nearest integer is n (n is an integer of from 0 to 14), in the case where n is in the range of from 3 to 10, the pH of a stable composition for external use is in the range of n $\pm$ 2, and the pH may be adjusted in the range of from 3 to 10. In addition, when the rounded numeric value of the pH of a 0.5% by weight aqueous solution of the bulk product of a fullerene, a fullerene derivative, a fullerene-modified compound, a clathrate compound of a fullerene or a salt thereof at 20˚C is n, in the case where n is 3 or less, the pH of a stable fullerene preparation for external use may be adjusted in the range of from 3 to 4, and in the case where the pH of n is 10 or more, the pH may be adjusted in the range of from 9 to 10. In any case, it is preferred that the pH of a stable fullerene-containing composition for external use is adjusted in the range of from 3 to 10.

Examples of the indication for which the preventive/ therapeutic composition for a free radical disease of the present invention can exhibit its effect include the following free radical diseases in which free radical is directly or indirectly associated with a part of the cause thereof. Specifically, the free radical disease is myocardial infarction, an ischemic heart disease, heart failure, angina pectoris, cardiac arrhythmia, arteriosclerosis, disturbance of lipid metabolism in the liver, hyperlipemia, essential hypertension, hypertension, arteriosclerosis, coronary arteriosclerosis, thrombosis, arteriosclerosis obliterans, vascular disorder, peripheral vascular disorder, cholestasis, hypercholesterolemia, pancreatic damage, organ failure, acute or chronic hepatitis, gastric ulcer, duodenal ulcer, colitis ulcerosa, digestive system damage, cholecystopathy, diabetes, arthritis therapeutic agent, rheumatoid, liver failure, liver damage, ischemic liver damage, disturbance of lipid metabolism in the liver, gallbladder damage, organ transplantation damage, diabetes, toxicosis, organ transplantation. damage, cancer, ischemic reperfusion damage, tissue aging, skin pigmentation, skin wrinkle, skin seborrhea, skin tanning, skin acne, burn injury, obesity, hair loss, mental disorder, dementia, Parkinson's disease, an infectious disease or the like.

As described above, the preventive/therapeutic composition for a free radical disease of the present invention is also useful for the treatment and prevention of a mammal (for example, a mouse, a rat, a pig, a raccoon dog, a fox, a cat, a house rabbit, a dog, a cow, a horse, a goat, a monkey or a human) having cancer (tumor), and has a significant effect on prolonging the life and suppressing cancer metastasis of such a tumor-bearing animal. Specific examples of the cancer which is the indication for the preventive/therapeutic composition for a free radical disease of the present invention include a tumor, a benign tumor or a malignant tumor.

Further, examples thereof also include malignant melanoma, malignant lymphoma, gastrointestinal cancer, lung cancer, esophageal cancer, stomach cancer, large bowel cancer, rectal cancer, colon cancer, ureteral tumor, gallbladder cancer, bile duct cancer, biliary tract cancer, breast cancer, liver cancer, pancreatic cancer, testicle tumor, maxillary cancer, tongue cancer, lip cancer, oral cavity cancer, pharyngeal cancer, larynx cancer, ovary cancer, uterine cancer, prostate cancer, thyroid cancer, brain tumor, Kaposi's sarcoma, hemangioma, leukemia, polycythemia vera, neuroblastoma, retinoblastoma, myeloma, bladder tumor, sarcoma, osteogenic sarcoma and myosarcoma, and further include skin cancer, basal cell cancer, skin appendage carcinoma, skin metastasis cancer, skin melanoma and the like.

At this time, the preventive/therapeutic composition for a free radical disease of the present invention also exhibits an effect on the treatment of cancer and the prevention of cancer (also including inhibition of metastasis), therefore, by further incorporating at least one kind of antitumor agent therein, the effect on the treatment of cancer and the prevention of cancer (also including inhibition of metastasis) can be further enhanced. As the antitumor agent, for example, nitromin (R), cyclophosphamide, merphalan, thiotepa, carboquone, Protecton (R), busulfan, nimustine hydrochloride, mitobronitol, ifosfamide, mercaptopurine, thioinosine, cytarabine, dacarbazine, fluorouracil, tegaful, ancitabine hydrochloride, methotrexate, carmofur, UFT (R), enocitabine, vinblastine sulfate, vincristine sulfate, vindesine sulfate, actinomycin (D), mitomycin C, chromomycin A3, bleomycin hydrochloride, bleomycin sulfate, daunorubicin hydrochloride, doxorubicin hydrochloride, neocarzinostatin, peplomycin sulfate, aclarubicin hydrochloride, mepitiostane, epitiostanol, tamoxifen citrate, Honvan, Picibanil (R), krestin, lentinan, L-asparaginase, aceglatone, procarbazine hydrochloride, floxuridine, MDS KOWA 3000 (R), cisplatin, Estracyt (R), Sizofiran, protamine, an angiostatic steroid in the presence of heparin, an antitumor polysaccharide, a laminin peptide, an antitumor polypeptide containing an Arg-Gly-Asp (RGD) sequence, an antitumor platelet factor or the like can be used.

Further, an effect on suppressing cancer metastasis can be enhanced also in the case where the active ingredient of the present invention is used in combination with an antitumor agent which has been reported recently, protamine, an angiostatic steroid in the presence of heparin, a polysaccharide such as a peptideglycan complex, a laminin peptide such as Cys-Asp-Pro-Gly-Tyr-Ile- Gly-Ser-Arg-NH2 (CDPGYIGSR-NH2), a peptide containing an Arg-Gly-Asp (RGD) sequence, a platelet factor-4, or an anticancer agent such as an interferon obtained from a natural source or by a genetic engineering technique.

Further, when the above-mentioned currently available cancer metastasis inhibitor is administered in vivo, in the case of a human, side effects such as leukopenia, thrombopenia, bleeding, anemia, anorexia, nausea, vomiting, stomatitis, diarrhea, skin rash, hair loss, skin pigmentation, fever, malaise, headache, liver function failure, proteinuria, edema or hypersensitivity are observed in some cases. However, when the fullerene, which is the active ingredient of the invention of this application, is used in combination, these side effects are alleviated.

Further, specific examples of the ischemic reperfusion damage which is the indication for the preventive/ therapeutic composition for a free radical disease of the present invention include an ischemic heart disease, ischemic reperfusion myocardial damage, ischemic liver damage, ischemic reperfusion liver damage, ischemic reperfusion renal damage, ischemic reperfusion pancreatic damage, ischemic reperfusion gallbladder damage, ischemic reperfusion cardiovascular damage, ischemic reperfusion gastrointestinal damage, ischemic reperfusion muscle damage, ischemic reperfusion vascular damage, ischemic reperfusion eye damage and ischemic reperfusion skin damage.

Further, examples of the infectious disease which is the indication for the preventive/therapeutic composition' for a free radical disease of the present invention include a bacterial infectious disease such as an enterobacterial infectious disease caused by E. coli or the like, a viral infectious disease such as hepatitis caused by a hepatitis virus, acquired immunodeficiency syndrome (AIDS) caused by HIV, common cold caused by rhinovirus, or influenza caused by influenza virus, a fungal infectious disease such as candidiasis caused by a fungus of the genus Candida and a parasitic infectious disease such as malaria caused by Plasmodium.

It has been reported that these diseases or the diseases described below are caused by free radical or one of the causative factors thereof is free radical in vivo in "Kassei Sanso to Byotai (Free Radical and Pathologic Conditions)" edited by Inoue, Japan Scientific Societies Press, 1992, "Kosanka Busshitsu (Antioxidants)" edited by Niki, et al., Japan Scientific Societies Press, 1994 and "Gendai Iryo (Modern Medicine)" vol. 25, No. 10, 1993, and references described in these books and the like.

As an example of the free radical diseases to be a target for the preventive/therapeutic composition for a free radical disease of the present invention, there are organ failure and tissue damage at the time of organ transplantation to be carried out for tissue exchange. To be more specific, organ damage caused by arresting or slowing the blood flow flowing in cells or tissues due to the surgery for transplantation of an organ such as heart, liver, kidney, pancreas, gallbladder, thymus, stomach, lung, intestine or skin, the vascular surgery such as coronary artery recanalization surgery and the bypass surgery thereof, and diseases and events described below, damage or death of cells or tissues or damage accompanying organ failure which occurs during or after the reperfusion after the arrest of the blood flow and the like can be exemplified, and the composition of the present invention also has an effect on these diseases. Further, as other free radical diseases to be a target for the preventive/ therapeutic composition for a free radical disease of the present invention, damage or death of cells or tissues, diseases and failure of organs including vessels and the like accompanying a physical decrease in the blood flow rate or arrest of the blood flow caused by thrombosis of vessels attached to the above-mentioned organs to be a target in the present invention, anemia, ischemia, vascular sclerosis, vasoconstriction, bleeding (Obayashi, Proc. Soc. Exp. Biol. Wed., 196, 196, 164-169, 1990) or the like can be exemplified, and the composition of the present invention also has an effect on these.

Further, as other free radical diseases to be a target for the preventive/therapeutic composition for a free radical disease of the present invention, oxygen deficiency caused by a decrease in oxygen pressure in vessels, oxygen deficiency damage in cells or tissues caused by a chemical substance such as agrochemical or carbon monoxide

intoxication, temporary oxygen deficiency in tissues and the like can also be included, and damage or death of cells or tissues, diseases and failure of organs including vessels and the like observed when blood is sent again to cells or tissues in a normal or close to normal condition by an appropriate treatment such as blood transfusion or an event thereafter can be exemplified, and the composition of the present invention also has an effect on these.

Further, specific examples of an ischemic reperfusion disease of a free radical disease which is to be a target for the preventive/therapeutic composition for a free radical disease of the present invention and on which the composition particularly has an effect include ischemic reperfusion damage such as ischemic reperfusion myocardial damage (Narita, W. J. J., Lab. Clin. Med., 110, 153-158, 1987, Smith, L. L. Phil. Trans. R. Soc. Lond., 311, 647-657, 1985), ischemic reperfusion liver damage (Inoue, M. Inmucosal Immunology, 527-530, Elsevier, Amsterdam, 1990, Nakahama, Kanzo (Liver), 32: 1110-1123, 1991, Takekawa, Kanzo (Liver), 30: 459-467, 1989, Shirosugi, Nippon Shokaki Geka Gakkai Zasshi (Japanese Journal of Gastroenterological. Surgery), 26: 358, 1993) ischemic reperfusion renal damage, ischemic reperfusion pancreatic damage (Isaji, S. : Mie Med. J. 35: 109-123, 1985), ischemic reperfusion gallbladder damage (Taoka, Gastroent. JPN., 26: 653-644, 1991), ischemic reperfusion cardiovascular damage, ischemic reperfusion gastrointestinal damage (Iwai, Nippon Shokaki Geka Gakkai Zasshi (Japanese Journal of Gastroenterological. Surgery), 87: 1662-1669, 1990, Naito, Y., Free Red. Res. Comms., 16: 13. 5, 1992), ischemic reperfusion muscle damage, ischemic reperfusion vascular damage, ischemic reperfusion eye damage and ischemic reperfusion skin damage, and the composition of the present invention has an effect on both the prevention and treatment thereof.

Further, the preventive/therapeutic composition for a free radical disease of the present invention has an effect also on various damages induced by free radical as described below. Specific examples thereof include Behcet's disease, radiation damage, adverse effects of an antitumor agent, bacterial shock, cachexia, autoimmune diseases, burn injury, herpesvirus, adult T-cell leukemia, thioredoxin syndrome, traumatic shock, amyotrophic lateral sclerosis, asexual myocardial infarction and the like.

The preventive/therapeutic composition for a free radical disease of the present invention has low toxicity and can be orally or parenterally administered to a mammal including a human.

The form of the preventive/therapeutic composition for a free radical disease of the present invention is not particularly limited, and examples of the form thereof include a preparation for oral administration, a liquid for infusion, a tablet, a powder, a liquid suppository, a preparation for external use, an ointment, an adhesive preparation, an eye drop, an intravenous injection, a powdered drug, a granule, a tablet, a sugar-coated tablet, a capsule, a pill, a suspension, a liquid, an ampule, an injection, an isotonic solution and the like, and further, it can also be applied to a pharmaceutical product in any other form. The main active ingredient is a fullerene, and examples thereof include a cyclodextrin clathrate of a C60 fullerene, hydroxylated C60 and C70 fullerenes in which hydroxyl groups are directly linked at a hydroxylation rate of about 50/mol of fullerene or more, and an alkali metal salt thereof. Examples of the alkali metal salt include a sodium salt, a potassium salt, a calcium salt, a magnesium salt and the like.

The preventive/therapeutic composition for a free radical disease of the present invention may be formulated as a mixture with a pharmaceutically acceptable inert carrier or a diluent, and/or another pharmacologically active substance, or may be formulated into a dose unit form.

Further, the preventive/therapeutic composition for a free radical disease of the present invention may be formulated as a complex in accordance with a known pharmaceutical production process. For example, for the purpose of promoting the absorption thereof by increasing the solubility in water and increasing the pharmacological activity, the main ingredients of the present invention may be used as a complex with cyclodextrin or maltosyl-cyclodextrin.

The preventive/therapeutic composition for a free radical disease of the present invention is generally used orally or parenterally as a pharmaceutical composition prepared by mixing such an active ingredient with a pharmacologically acceptable carrier or excipient.

Examples of the form thereof include the one obtained by preparing the respective active ingredients as an aqueous solution in advance, the one obtained by lyophilizing the respective active ingredients and preparing them as a solid mixture, the one obtained by preparing the respective active ingredients as aqueous solutions and lyophilizing the resulting aqueous solutions thereby to form solid matters separately, the one obtained by preparing any of the active ingredients as an aqueous solution and lyophilizing the other active ingredients thereby to form a solid matter, a kit obtained by separately formulating the respective active ingredients into preparations and the like.

In the present invention, these active ingredients can be mixed and administered as a one-part preparation in accordance with a known pharmaceutical production process using, if desired, a pharmaceutically acceptable diluent or excipient described in Japanese Pharmacopoeia, 12th ed., (1991) (Hirokawa Shoten) or the like.

Further, the respective active ingredients are separately formulated into preparations, if desired, using a pharmaceutically acceptable diluent, excipient or the like and can be administered as a one-part preparation by using a diluent or the like immediately before use. Further, the composition can be formulated into a dosage form in which the separately formulated respective preparations are prepared as a kit as described above and can be administered to the same subject separately, simultaneously, or at a predetermined time interval through the same route or different routes.

In the case where the composition of the present invention is a solution, it can be prepared by an ordinary

method using a solvent such as a water-soluble solvent (for example, distilled water or the like), a water-soluble preparation (for example, a physiological saline solution, a Ringer's solution or the like) or an oily solvent (for example, sesame oil, corn oil, olive oil or the like). At this time, if desired, an additive such as a solubilizing agent (for example, sodium salicylate, sodium acetate or the like), a buffer (for example, sodium citrate, glycerin or the like), a tonicity agent (for example, glucose or the like), a stabilizer (for example, human serum albumin, polyethylene glycol or the like), a preservative (for example, benzyl alcohol, phenol or the like) or a soothing agent (for example, benzalkonium chloride, procaine hydrochloride or the like) can also be used.

Further, the composition of the present invention can be formulated, if desired, by mixing or using a pharmacologically or pharmaceutically acceptable additive (for example, a diluent, an excipient, a binder, a disintegrant, a colorant, a stabilizer, an extender, a wetting agent, a surfactant, a lubricant, a dispersant, a buffer, a flavor, a fragrance, a perfume, a preservative, a solubilizing agent, a solvent, a coating agent, a sugar-coating agent or the like) and the resulting preparation can be used.

The diluent to be used in the preparation of the preventive/therapeutic composition for a free radical disease of the present invention is a pharmaceutically acceptable diluent. The diluent is a material other than the compound of the invention of this application and may be in the form of a solid, a semisolid, a liquid or an ingestible capsule, and various materials can be exemplified.

For example, the preventive/therapeutic composition for a free radical disease obtained according to the present invention may be produced by any conventionally known method. For example, the active ingredient is mixed with a diluent and formulated into, for example a granule, then, the composition thereof can be also formed into, for example, a tablet.

A preparation for parenteral administration should be sterile, or if desired, should be isotonic with the blood. The parenteral administration includes administration by means of injection (including, for example, intravenous muscular injection infusion) and rectal administration (suppository).

The composition of the present invention can be a cancer metastasis inhibitor as it is, therefore, the fullerene or a salt thereof which is an active ingredient is preferably contained in the preparation and the composition in an amount of from 0.001 to 100% by weight in general.

Examples of the composition for oral administration further include a tablet, a pill, a granule, a powder, a capsule, a syrup, an emulsion, a suspension, a propellant and the like. Such a composition is produced by a known method and lactose, starch, sucrose, magnesium stearate or the like is used as a carrier or an excipient.

For parenteral administration, for example, the composition of the present invention can be formulated into an injection, a suppository, an adhesive agent, an eye drop, a preparation for external use or the like. As the injection, for example, the composition of the present invention can be used by formulating it into an intravenous injection, a subcutaneous injection, an intramuscular injection, an injection for infusion or the like. The injection is generally provided by filled in an appropriate ampule. As the dosage form, for example, an endorectal suppository, a vaginal suppository and the like can be exemplified. As the preparation for external use; an ointment, a preparation for nasal administration, a preparation for percutaneous administration and the like can be exemplified.

In order to obtain a preparation for external use, for example, the composition of the invention of this application can be formulated into a solid; semisolid or liquid preparation for external use in accordance with a known method. For example, in the case of the solid preparation for external use, the composition of the invention of this application is processed into a powder composition as such or after adding and mixing thereto an excipient (for example, glycol, mannitol, starch, a microcrystal, cellulose or the like), a thickener (for example, a natural gum, a cellulose derivative, an acrylic polymer or the like) or the like.

In the case of the liquid preparation for external use, almost similar to the case of an injection, the composition of the present invention is formulated into an oily or aqueous suspension. In the case of the semisolid preparation for external use, an aqueous or oily gel or the one in the form of an ointment is preferred.

Further, in any case, a pH adjusting agent (for example, carbonic acid, a monoester, citric acid, hydrochloric acid, sodium hydroxide or the like), an antiseptic (for example, a p-hydroxybenzoic acid ester, chlorobutanol, benzalkonium chloride or the like) or the like may be added. In order to obtain a suppository, the composition of the invention of this application can be formulated into an oily or aqueous solid, semisolid or liquid suppository in accordance with a known method.

The diameter of the molecular cluster of the fullerene in a solution such as an injection of the preventive/therapeutic composition for a free radical disease of the present invention is set to 10 nm or less, whereby a uniform dissociative solution can be obtained. By doing this, it was found that a risk of arrest of the blood flow in capillary vessels can be avoided. As a result, the preventive/therapeutic composition for a free radical disease including the fullerene of the present invention has low toxicity showing an $LD_{50}$ value of 1000 mg/kg or more in an acute toxicity test using a mouse, a rat and a beagle dog, and further, in a subacute toxicity test using a rat, abnormality was not observed even when the composition of the present invention was administered at a dose of up to 500 mg/kg/day. From these results, it was confirmed that the composition of the present invention has high safety.

Further, the concentration of the fullerene to be blended in the composition of the present invention is 0.01 to 1000 ppm based on the total weight of the preventive/ therapeutic composition for a free radical disease of the present invention, however, when a favorable solubility in the blood is considered, it is preferably 0.1 to 100 ppm.

As a substance that can be blended in the preventive/ therapeutic composition for a free radical disease of the present invention for the purpose of being used as a stabilizer, an effect activator or the like, for example, one kind or a mixture of two or more kinds selected from water-soluble high molecular weight compounds such as metrazol, etoposide, cholic acids, dimethylsulphoxide, adenosine phosphates, polyethylene glycols and polybutylene glycols; inorganic salts such as sodium chloride, potassium chloride and magnesium chloride; organic acids such as citric acid, malic acid, phosphoric acid, edetic acid, oxalic acid, lactic acid, butyric acid, acetic acid, ascorbic acid, erythorbic acid, sialic acid and amino acid; salts of these organic acids; polyhydric alcohols such as propylene glycol, butylene glycol and glycerin, which are generally used as an additive, can be exemplified.

The cholic acid is selected from bile acid, dehydrocholic acid, deoxycholic acid, and their salts of alkaline earth metals such as sodium and potassium. The adenosine phosphate is selected from adenosine phosphoric acid esters such as adenosine 5'-triphosphate, adenosine 5'-diphosphate and adenylic acid.

As the dosage form of the preventive/therapeutic composition for a free radical disease of the present invention, from the viewpoint that the administration method is simple and the effect is high, a preparation for oral administration, a preparation for external use and an injection are preferred. With regard to the pharmaceutical agent of the present invention, the above-mentioned pure substance or a mixture thereof can be formulated into a preparation by combining it with a known carrier for pharmaceutical use, and an administration method by intravenous injection of an injectable preparation or an infusion preparation is preferred because the highest effect can be achieved and the administration method is simple.

Here, when it is considered that the composition of the invention is used as an injectable preparation or a preparation for oral administration, the one with a high solubility in water is preferred. Therefore, a water adduct or a crystallization water adduct of a fullerene is useful because it has a higher solubility in water than that of an anhydride thereof.

The content of water or crystallization water in the water adduct or crystallization water adduct of a fullerene of the present invention is not particularly limited. However, in order to keep a better solubility, a water adduct or a crystallization water adduct of a fullerene that keeps the water content of from 1 to 50% by weight, more preferably from 5 to 20% by weight in the case of a water adduct, and the water molecules of from 1 to 20 molecules, more preferably from 1 to 10 molecules per fullerene molecule in the case of a crystallization water adduct is preferred.

The dose of the fullerene of the present invention varies depending on the symptom, age, sex, body weight, dosage form or administration route. However, for example, in the case of oral administration, suppository administration, external application or the like, the dose is generally in the range of from 0.001 to 8500 mg, preferably from 1 to 100 mg for an adult per 1 kg of the body weight per day, and in the case of intravenous injection or infusion, it is generally in the range of from 0.025 to 200 mg, preferably from 0.25 to 100 mg for an adult per 1 kg of the body weight per day. The preparation may be administered at once or by dividing the dose into several times.

Further, in the case where the pharmaceutical agent of the present invention is administered by intravenous injection, the injectable solution of the present invention is intravenously injected or dripped into the vein or the like in an amount of from 0.1 to 50 cc per 1 kg of the body weight of a human such that the rate of the total amount of the substances dissolved in the injectable solution becomes not more than 1 g/kg/h. In the case where the total amount of the substances dissolved in the injectable solution is a high concentration of 20% or more, the administration can be carried out by starting with a solution with a low concentration and increasing the concentration thereof gradually. The dose of the fullerene of the present invention varies also depending on the type of preparation or the administration method, however, it is administered generally at a dose of 1 $\mu$mg to 10 mg per 1 kg of the body weight per day at once or by dividing the dose into several times. Further, in particular, at the time of severe ischemic reperfusion during surgery accompanying organ transplantation, cardiovascular bypass surgery or the like, and at the time of reperfusion after severe anemia, hypotension, cardiac arrest, oxygen deficiency or the like, and at the time of systemic ischemic reperfusion such as reoxygenation, by administering the fullerene and cleaved fullerene of the present invention and a salt of derivative thereof at a dose of 100 mg or more per 1 kg of the body weight per day, a larger effect can be expected.

With regard to the preventive/therapeutic composition for a free radical disease of the present invention, by adjusting the type and amount of the fullerene and cleaved fullerene of the present invention and a salt of derivative thereof and adding the resulting matter, or by adding an additive that can adjust the osmotic pressure to the fullerene and cleaved fullerene of the present invention and a salt of derivative thereof, the permeability of the fullerene and cleaved fullerene of the present invention and the salt of derivative thereof, which are the main ingredients of a liquid preparation to tissues or cells is increased, whereby a larger effect can be obtained. The additive that can adjust the osmotic pressure is not particularly limited as long as it is a physiologically acceptable additive such as one member or a complex of two of more members selected from inorganic salts such as sodium chloride and potassium chloride; organic acids such as ATP, malic acid and citric acid; salts of organic acids such as a sodium salt thereof; saccharides

such as glucose and fructose and the like.

By adding an antioxidant to the preventive/ therapeutic composition for a free radical disease of the present invention, an effect thereof on treating or preventing an adult disease can be enhanced. As the antioxidant, one member or a mixture of two or more members selected from vitamin B, L-ascorbic acid, tocopherol, L-ascorbic acid-2-phosphate and other antioxidative vitamin derivatives such as saccharo-L-ascorbic acid, ubiquinone, uric acid, cysteine, glutathione, glutathione peroxidase, SOD, citric acids, phosphoric acids, polyphenols, sodium bisulfite, sodium sulfite, erythorbic acid, sodium erythorbate, dilauryl thiodipropionate, tocotrienol, lipoic acid, tolylbiguanide, nordihydroguaiaretic acid, parahydroxy anisole, butylhydroxy anisole, dibutylhydroxy toluene, ascorbyl stearate, ascorbyl palmitate, octyl gallate, propyl gallate, carotenoid, flavonoid, tannin, lignin, saponin apple extract and clove extract, nucleic acids, Chinese herbal medicines, seaweeds, inorganic compounds, and conventionally available antioxidants described to have an antioxidative function in Japanese Pharmacopoeia can be added in combination.

The fullerene which is the active ingredient of the preventive/therapeutic composition for a free radical disease of the present invention inhibits the oxidative degradation of such an antioxidant, particularly ascorbic acid and stabilizes it, therefore, the degradation of a preparation over time does not almost occur during the production of the preparation, storage period and in the gastrointestinal tract. Further, when common ascorbic acid is incorporated in vivo, it is degraded by particularly a radical contained in a food or the like present in the gastrointestinal tract and it is hydrolyzed and loses its activity in vivo before it reaches a target cell, however, because the fullerene which is the active ingredient of the invention of this application is stable, it is absorbed by nutrient absorbing cells or the like in vivo without losing its activity.

Further, according to the results of the study performed by the present inventors, a fullerene shows an extremely high cell absorption property. It has been confirmed that because of this property, the intracellular concentration of ascorbic acid can be increased by about up to 30 times compared with a fullerene, and by about up to 2 times compared with ascorbic acid. It is presumed that as described above, the preventive/therapeutic composition for a free radical disease of the present invention can exhibit a cancer metastasis inhibitory action that cannot be observed in ascorbic acid.

Further, it has been confirmed that a fullerene exhibits a cancer metastasis inhibitory action by a new mechanism that has not been reported so far. In general, a cancer cell has a migratory property and accordingly, penetrates from the focus to the blood vessel and can migrate. As a result, the cancer cell metastasizes to a wide range of organs and proliferates there. Due to this, the period of life extension is considerably shortened.

On the other hand, with regard to the antitumor action of the fullerene of the present invention, it has been confirmed that the cancer metastasis inhibitory action is exhibited by particularly inhibiting the migratory property of a cancer cell from the focus to the vascular endothelium thereby to inhibit the metastasis of cancer. It was found that a cancer cell to which the fullerene of the invention of this application was administered showed an extremely low invasion rate into the vascular endothelium. Further, it has been confirmed that this action is an action unique to the fullerene.

As described above, the fullerene which is the active ingredient of the preventive/therapeutic composition for a free radical disease of the present invention inhibits the oxidative degradation of ascorbic acid and stabilizes it. Further, the fullerene exhibits an extremely high cell absorption property and can efficiently increase the intracellular concentration of ascorbic acid, therefore, the fullerene allows the antitumor action of ascorbic acid to be exhibited maximally. It is considered that with respect to the antitumor action of the fullerene, the antitumor action is exhibited by particularly inhibiting the invasion of a tumor cell into the vascular endothelium thereby to inhibit the metastasis of cancer.

The preventive/therapeutic composition for a free radical disease of the present invention can be applied to a pharmaceutical product, a quasi drug, a cosmetic or a functional food. The dosage form of the composition of the present invention can be any such as a powder, a liquid, a tablet, a gel, an emulsion or a capsule, and specifically, it can be applied to an oral preparation, an injection, an ointment, a suppository, a preparation for external use or the like. More specifically, it can be used as a product such as a lotion, a milk lotion, a cream, a facial mask, a shampoo, a rinse, a hair restoration agent, a hair growth agent, a hair dressing product, a toothpaste, a gargle or a bath agent, and it is not particularly limited.

As the cosmetic in the present invention, for example, it is classified into a make-up cosmetic such as a foundation, a face powder, an eye shadow, an eyeliner, an eyebrow pencil, a rouge, a lipstick or a nail varnish; a basic skin care product such as an emulsion, a cream, a lotion, a calamine lotion, a sunscreen agent, a suntan agent, an after-shave lotion, a pre-shave lotion, a facial mask, an anti-acne product or an essence; a hair cosmetic product such as a shampoo, a rinse, a conditioner, a hair dye, a hair tonic, a hair setting agent, a hair growth agent or a permanent agent; a body powder, a deodorant, a hair removal agent, a soap, a body shampoo, a bath agent, a hand soap, a perfume and the like, and the composition of the present invention can be used as any cosmetic.

The form of the formulation of the present invention is not particularly limited, and a conventionally known form such as a double layer, an oil-in-water emulsion, a water-in-oil emulsion, a gel, a spray, a mousse, an oil, a solid, a sheet or a powder can be employed.

As described above, the present invention could provide a preventive/therapeutic composition for a free radical disease, which is stable during the production process and storage, has less side effects on the living body, and is safe and effective by incorporating a fullerene having a high radical scavenging effect in vivo as a main component of an

active ingredient.

Examples

Hereinafter, the present invention will be described with reference to Examples and Comparative examples. It is a matter of course that the present invention is not limited to these examples.

A: Effect of preventive/therapeutic composition for a free radical disease on various diseases

A-1: Production of composition

Example 1: Injectable solution A

A mixture obtained by uniformly mixing a cyclodextrin clathrate of a' C60 fullerene and a cyclodextrin clathrate of a C70 fullerene at a weight ratio of 8:2 was completely dissolved in 1000 cc of a Ringer's solution for pharmaceutical use at a concentration of 178 ppm, and the resulting solution was sterilized by filtration with a sterilization filter for injection production, whereby an injectable solution was produced as a preventive/ therapeutic agent for a free radical disease for intravenous injection of the present invention.

Example 2: Injectable solution B

A mixture obtained by uniformly mixing a hydroxylated C60 fullerene with a hydroxylation rate of 87% (by mole) and a hydroxylated C70 fullerene with a hydroxylation rate of 61% (by mole) at a weight ratio of 8:2 was completely dissolved in 1000 cc of a Ringer's solution for pharmaceutical use at a concentration of 5 ppm, and the resulting solution was sterilized by filtration with a sterilization filter for injection production, whereby an injectable solution was produced as a preventive/ therapeutic agent for a free radical disease for intravenous injection of the present invention.

Example 3: Injectable solution C

A fullerene of Radical sponge (trade name) (with a fullerene concentration of 200 ppm) manufactured by Vitamin C60 BioResearch Corporation was completely dissolved in 1000 cc of a Ringer's solution for pharmaceutical use at a concentration of 1%, and the resulting solution was sterilized by filtration with a sterilization filter for injection production, whereby an injectable solution was produced as a preventive/ therapeutic agent for a free radical disease for intravenous injection of the present invention.

Example 4: Injectable solution D

110 ppm of a PVP/fullerene complex in which 11 types of fullerene derivatives manufactured by Vitamin C60 BioResearch Corporation, C60, C70, $C_{120}$, $(C_{59}N)_2$, $C_{180}$, $C_{62}$, $C_{60}F$, $C_{60}F_{18}$, an open-cage fullerene, a fullerene with holes and corannulene, were blended in an amount of 10 ppm each was completely dissolved in 1000 cc of a Ringer's solution for pharmaceutical use at a concentration of 1%, and the resulting solution was sterilized by filtration with a sterilization filter for injection production, whereby an injectable solution was produced as a preventive/therapeutic agent for a free radical disease for intravenous injection of the present invention.

Example 5: Water-soluble preparation for external use and cosmetic lotion

A complex of fullerenes obtained by mixing 25% by weight of a mixture obtained by uniformly mixing a cyclodextrin clathrate of a C60 fullerene and a cyclodextrin clathrate of a C70 fullerene at a weight ratio of 8:2, 25% by weight of a mixture obtained by uniformly mixing a hydroxylated C60 fullerene with a hydroxylation rate of 87% (by mole) and a hydroxylated C70 fullerene with a hydroxylation rate of 61% (by mole) at a weight ratio of 8:2 and 50% by weight of a fullerene of Radical sponge (trade name) (with a fullerene concentration of 200 ppm) manufactured by Vitamin C60 BioResearch Corporation was dispersed and dissolved at 1% by weight in 90% by weight of purified water, 4% by weight of glycerin and 5% by weight of butylene glycol, whereby a water-soluble preparation for external use and a cosmetic lotion containing fullerenes were produced.

Example 6: Powder for oral administration

A complex of fullerenes obtained by mixing 25% by weight of a mixture obtained by uniformly mixing a cyclodextrin clathrate of a C60 fullerene and a cyclodextrin clathrate of a C70 fullerene at a weight ratio of 8:2, 25% by weight of a mixture obtained by uniformly mixing a hydroxylated C60 fullerene with a hydroxylation rate of 87% (by mole) and a hydroxylated C70 fullerene with a hydroxylation rate of 61 % (by mole) at a weight ratio of 8:2, 45% by weight of a fullerene of Radical sponge (trade name) (with a fullerene concentration of 200 ppm) manufactured by Vitamin C60 BioResearch Corporation and 5% by weight of corannulene ($C_{20}H_{10}$) was diluted with mannitol at a concentration of 1% by weight, whereby a powder for oral administration for preventing or treating myocardial infarction, heart failure, angina pectoris, cardiac arrhythmia, arteriosclerosis, disturbance of lipid metabolism in the liver, hyperlipemia, essential hypertension, hypertension, arteriosclerosis, coronary arteriosclerosis, thrombosis, arteriosclerosis obliterans, vascular disorder, peripheral vascular disorder, cholestasis, hypercholesterolemia, pancreatic damage, organ failure, acute or chronic hepatitis, gastric ulcer, duodenal ulcer, colitis ulcerosa, digestive system damage, cholecystopathy, diabetes, arthritis therapeutic agent, rheumatoid, liver failure, liver damage, disturbance of lipid metabolism in the liver, gallbladder damage, organ transplantation damage, diabetes, toxicosis, organ transplantation damage, cancer, tissue aging, skin pigmentation, skin wrinkle, alveolar pyorrhea, skin seborrhea, skin tanning, skin acne, burn injury, obesity, hair loss, mental disorder, dementia, Parkinson's disease, AIDS caused by HIV, common cold or influenza.

A-2: Evaluation

(Effect test)

By using injectable solutions in the test group including the preventive/therapeutic agent for a free radical disease of the above-mentioned Examples and in a control group (only a Ringer's solution), the effect of the pharmaceutical composition of the present invention was confirmed by comparison based on the following tests.

1) Test for effect on heart disease

A rat was subjected to thoracotomy and the left anterior descending artery was occluded for 15 minutes, followed by reperfusion. In a test group, the pharmaceutical composition of Example 1 of the present invention was intravenously administered to the rat at 5 mg/kg at 15 minutes before the occlusion of the coronary artery in advance, and in a control group, only a Ringer's solution was injected in the same manner as in the test group. When the level of free radical-dependent chemiluminescence in the peripheral blood was measured using a chemical luminescence from the start of this experiment, a significant increase in the level of free radical was observed from immediately after the initiation of reperfusion. Although an apparent cardiac arrhythmia was not observed on the electrocardiogram monitor during the occlusion of the coronary artery, after the initiation of reperfusion, within several seconds, an apparent cardiac arrhythmia, which is an indicator of heart disease was developed only in the control group. On the other hand, in the group in which the injectable solution of Example 1 of the present invention was intravenously administered, a cardiac arrhythmia observed in the control group was clearly suppressed, therefore, the preventive/ therapeutic composition for a free radical disease of the present invention was proved to have an effect on a heart disease.

2) Test for effect on liver damage

The portal vein and coronary artery of a rat were occluded for 20 minutes, followed by reperfusion for 60 minutes. Then, the liver was fixed by perfusion with glutaraldehyde and observed using an electron microscope. Further, the level of free radical-dependent chemiluminescence in the peripheral blood was measured using a chemical luminescence from the start of this experiment, and a significant increase in the level of free radical was observed from immediately after the initiation of reperfusion. In a test group, the injectable solution of Example 2 of the present invention was intravenously administered to the rat at 5 mg/kg at 15 minutes before the occlusion of the portal vein and coronary artery of the rat in advance, and in a control group, only a Ringer's solution was injected in the same manner as in the test group. As a result of observation using an electron microscope, in the control group, the spaces of Disse, spaces between the hepatocytes and the small bile ducts were significantly enlarged, a large number of vacuoles were observed in the cells, and pathologic conditions were clearly observed in the hepatocytes. Oh the other hand, in the observation using an electron microscope of the test group in which the injectable solution of the present invention was administered, the tissue structure of the liver was kept normal, therefore, the preventive/ therapeutic composition for a free radical disease of the present invention was proved to have an effect on a liver disease.

3) Test for effect on pancreatic disease

The coronary artery of a rat was occluded for 20 minutes, followed by reperfusion for 60 minutes. Then, a pancreatic disease model was produced, and the level of amylase in the blood was measured before the occlusion of

the coronary artery and at 4 hours after the initiation of reperfusion. When the level of free radical-dependent chemiluminescence in the peripheral blood was measured using a chemical luminescence from the start of this experiment, a significant increase in the level of free radical was observed from immediately after the initiation of reperfusion. In a test group, the injectable agent of Example 3, which is the preventive/therapeutic composition for a free radical disease of the present invention, was intravenously administered to the rat at 5 mg/kg at 15 minutes before the occlusion of the coronary artery of the rat in advance, and in a control group, only a Ringer's solution was injected in the same manner as in the test group. As a result of measurement of the level amylase in the blood, in the control group, the level of amylase in the blood was significantly higher than that before the occlusion, and a rat with the amylase level of up to 6000 U/dl was observed, and pathologic conditions of a pancreatic disease were clearly observed. On the other hand, the level of amylase in the blood in the test group in which the injectable agent of the present invention was administered was up to 1000 U/dl in the rat. Although a tendency of a slight increase in the level of amylase was observed compared with the normal condition before the occlusion, the level was significantly lower than that of the control group, therefore, the preventive/ therapeutic composition for a free radical disease of the present invention was proved to have an effect on a pancreatic disease.

4) Test for effect on kidney damage

The coronary artery of a rat was occluded for 20 minutes, followed by reperfusion for 60 minutes. Then, a kidney disease model in which damage was given to kidney tissues was produced, and the average level of protein in the urine was measured before the occlusion of the coronary artery and for 5 days after the initiation of reperfusion. When the level of free radical-dependent chemiluminescence in the peripheral blood was measured using a chemical luminescence from the start of this experiment, a significant increase in the level of free radical was observed from immediately after the initiation of reperfusion. In a test group, the injectable agent of Example 4 was intravenously administered to the rat at 5 mg/kg at 15 minutes before the occlusion of the coronary artery of the rat in advance, and in a control group, only a Ringer's solution was injected in the same manner as in the test group. As a result of determination of the average level of protein in the urine for 5 days after the initiation of reperfusion, in the control group, the level of protein in the urine was significantly higher than that before the occlusion, and pathologic conditions of a kidney disease were clearly observed. Oh the other hand, the level of protein in the urine in the test group in which the injectable agent of the present invention was administered was significantly lower than that of the control group, although a tendency of a slight increase in the level of protein in the urine was observed compared with the normal condition before the occlusion, therefore, the preventive/therapeutic composition for a free radical disease of the present invention was proved to have an effect on a kidney disease.

5) Test for effect on organ failure

A rat coronary artery occlusion model was subjected to systemic ischemia for 5 minutes, followed by reperfusion. When the level of free radical-dependent chemiluminescence in the peripheral blood was measured using a chemical luminescence manufactured by Showa Denko K. K. from the start of this experiment, a significant increase in the level of free radical was observed from immediately after the initiation of reperfusion. In a test group, the injectable agent of Example 3 was intravenously administered to the rat at 5 mg/kg at 15 minutes before the occlusion of the coronary artery of the rat in advance, and in a control group, only a Ringer's solution was injected in the same manner as in the test group. Also after the initiation of reperfusion, the agent for a disease associated with free radical of the present invention was injected at the same dose once daily for 5 days, and after 10 days, dissection was carried out, and cardiac, hepatic, renal, pancreatic and cystic cell necrosis indices were determined using a 45 Ca-autoradiography/image analyzer. In the rat in the control group, delayed cell death, in which a large number of cells of the respective organs died after the ischemia, was observed. On the other hand, it was confirmed that in the mouse (one group consisting of 10 mice) in which the injectable agent of the present invention was injected, necrosis of the respective cells was significantly prevented.

6) Test for comparative effect on organ failure

A rat coronary artery occlusion model was subjected to systemic ischemia for 5 minutes, followed by reperfusion. When the level of free radical-dependent chemiluminescence in the peripheral blood was measured using a chemical luminescence manufactured by Showa Denko K. K. from the start of this experiment, a significant increase in the level of free radical was observed from immediately after the initiation of reperfusion. In a test group, the injectable solution of Example 2 was intravenously administered to the rat at 5 mg/kg at 15 minutes before the occlusion of the coronary artery of the rat in advance, and in a control group, a Ringer's solution supplemented with human Cu-Zn SOD was injected in the same manner as in the test group. Also after the initiation of reperfusion, the above pharmaceutical

composition of the present invention was injected at the same dose once daily for 5 days, and after 10 days, dissection was carried out, and cardiac, hepatic, renal, pancreatic and cystic cell, necrosis indices were determined using a 45 Ca-autoradiography/image analyzer. In the rat in the control group, delayed cell death, in which a large number of cells of the respective organs died after the ischemia, was observed by carrying out observation with the 45 Ca-autoradiography/image analyzer. On the other hand, it was confirmed that in the mouse (one group consisting of 8 mice) in which the injectable solution of the present invention was injected, necrosis of the respective cells was significantly prevented.

7) Test for comparative effect on skin disease

In a total of 513 males and females suffering from pigmentation caused by sun exposure, skin wrinkle, oral alveolar pyorrhea, skin seborrhea, acne, burn injury, or hair loss, which is a skin disease considered to be associated with free radical, only mannitol was applied to 224 patients as a control group, and as a test group, the water-soluble preparation for external use of Example 5 was applied to the remaining 289 patients at a dose of 5 cc twice daily in the morning and in the evening (10 cc per day) for 3 months, or in the case of alveolar pyorrhea, the external preparation was allowed to stay in the mouse with a gargle for 3 minutes thereby to rinse the mouse, and the conditions were recorded every other week. As a result, a tendency of some sort of improvement was observed in 193 patients among the 289 patients in the test group, and an aggravating tendency was observed in 21 patients. On the other hand, in the 213 patients in the control group, a tendency of some sort of improvement was observed in 25 patients, and an aggravating tendency was observed in 19 patients. In the remaining patients, the pathologic conditions were not changed. Further, when the effectiveness in the respective pathologic conditions was statistically examined for the respective diseases, a therapeutic effect was observed similarly in the test group, however, a change was not observed in the control group. From these results, it was confirmed that the preventive/therapeutic agent for a free radical disease of the present invention has an effect on the above skin diseases. B: Effect of preventive/therapeutic composition for a free radical disease on cancer

B-1: Production of composition

Example 7: Tablet

By using the blended matters of fullerenes with a composition shown in Table 1, the preventive/therapeutic compositions for a free radical disease of the present invention shown in Table 2 were produced.

Table 1

| Formulation No. | Blending ratio (%) | | | | | | |
|---|---|---|---|---|---|---|---|
| | PVP/ fullerene (molecular weight of PVP: 60,000) | PVP/ fullerene (molecular weight of PVP: 40,000) | Polyhydroxylated fullerene | Na salt of polyhydroxylated fullerene | Fullerene polyester | Na salt of fullerene polyester | Na/K salt of fullerene polyester |
| 1 | 100 | | | | | | |
| 2 | | 100 | | | | | |
| 3 | | | 100 | | | | |
| 4 | | | 80 | 20 | | | |
| 5 | | | | | 100 | | |
| 6 | | | | | 50 | 50 | |
| 7 | | | | | | 60 | 40 |
| 8 | 50 | 50 | | | | | |
| 9 | 50 | | 30 | 20 | | | |
| 10 | | | 20 | 20 | 40 | 20 | |
| 11 | | | | 40 | 40 | 20 | |
| 12 | 50 | | | | 30 | 20 | |
| 13 | 50 | | | | 30 | | 20 |

Incidentally, PVP (polyvinylpyrrolidone)-modified conjugated fullerene: PVP/fullerene is the one prepared by, for example, the following process.

That is, first, a stirred solution obtained by dissolving mixed fullerenes (C60:C70 = 3.5:1 mol/mol) (0.8 mg) in toluene (1.0 ml) was added to a chloroform solution containing 100 mg of PVP (molecular weight: 60,000) at room temperature. After the solution was well mixed, the solvent was evaporated under a reduced pressure. The residue was dissolved in 2.0 ml of Milli-Q (registered trademark) water, and the resulting suspension was subjected to azeotropic distillation, whereby toluene was distilled off.

In this way, an aqueous solution of a fullerene/PVP complex was obtained. Further, water was removed by evaporation, whereby a powder was obtained.

Further, the polyhydroxylated fullerene shown in the table was the one in which 24 hydroxyl groups were linked to a fullerene molecule. The compound of the Na salt thereof is the one in which 4 hydroxyl groups of the linked hydroxyl groups were replaced with Na.

Further, the fullerene polyester was the one in which 2 hydroxyl groups of the hydroxylated fullerene were esterified with acetic acid, and the Na salt thereof was the one in which 2 hydroxyl groups were esterified with acetic acid and the other 2 hydroxyl groups were replaced with Na, and the Na/K salt thereof was the one in which 2 hydroxyl groups were esterified with acetic acid and the other 2 hydroxyl groups were replaced with Na and K.

Table 2

| Blending example No. | Concentration of hydroxymethyl cellulose | Component Type and blending concentration of active ingredient | |
|---|---|---|---|
| 1 | 40% by weight | Formulation No. 1 in Table 1 | 60% by weight |
| 2 | 40% by weight | Formulation No. 2 in Table 1 | 60% by weight |
| 3 | 40% by weight | Formulation No. 3 in Table 1 | 60% by weight |
| 4 | 40% by weight | Formulation No. 4 in Table 1 | 60% by weight |
| 5 | 40% by weight | Formulation No. 5 in Table 1 | 60% by weight |
| 6 | 40% by weight | Formulation No. 6 in Table 1 | 60% by weight |
| 7 | 40% by weight | Formulation No. 7 in Table 1 | 60% by weight |
| 8 | 40% by weight | Formulation No. 8 in Table 1 | 60% by weight |
| 9 | 40% by weight | Formulation No. 9 in Table 1 | 60% by weight |
| 10 | 40% by weight | Formulation No. 10 in Table 1 | 60% by weight |
| 11 | 40% by weight | Formulation No. 11 in Table 1 | 60% by weight |
| 12 | 40% by weight | Formulation No. 12 in Table 1 | 60% by weight |
| 13 | 40% by weight | Formulation No. 13 in Table 1 | 60% by weight |

A raw material obtained by well mixing the components of the formulation (% by weight) shown in the above Table 2 was tableted in accordance with a standard method using a common tabletting machine, whereby a tablet of the present invention was produced.

Example 8: Powder

The,components of the formulation (% by weight) shown in Table 3 were mixed and pulverized, whereby a powder of the present invention was produced.

Table 3

| Blending example No. | A mixture of butyl p-hydroxybenzoate, sodium citrate and citric acid at a weight ratio of 0.1 : 38 : 1.99 | Component Type and blending concentration of active ingredient | |
|---|---|---|---|
| 1 | 40% by weight | Formulation No. 1 in Table 1 | 60% by weight |
| 2 | 40% by weight | Formulation No. 2 in Table 1 | 60% by weight |
| 3 | 40% by weight | Formulation No. 3 in Table 1 | 60% by weight |
| 4 | 40% by weight | Formulation No. 4 in Table 1 | 60% by weight |
| 5 | 40% by weight | Formulation No. 5 in Table 1 | 60% by weight |

(continued)

| Blending example No. | A mixture of butyl p-hydroxybenzoate, sodium citrate and citric acid at a weight ratio of 0.1 : 38 : 1.99 | Component Type and blending concentration of active ingredient | |
|---|---|---|---|
| 6 | 40% by weight | Formulation No. 6 in Table 1 | 60% by weight |
| 7 | 40% by weight | Formulation No. 7 in Table 1 | 60% by weight |
| 8 | 40% by weight | Formulation No. 8 in Table 1 | 60% by weight |
| 9 | 40% by weight | Formulation No. 9 in Table 1 | 60% by weight |
| 10 | 40% by weight | Formulation No. 10 in Table 1 | 60% by weight |
| 11 | 40% by weight | Formulation No. 11 in Table 1 | 60% by weight |
| 12 | 40% by weight | Formulation No. 12 in Table 1 | 60% by weight |
| 13 | 40% by weight | Formulation No. 13 in Table 1 | 60% by weight |

Example 9: Injectable solution

1 g of each compound (formulation Nos. 1 to 10) shown in the above Table 1 subjected to a sterilization treatment was dissolved in 5% dextrose in water for injection in a sterile manner to give a final volume of 100 ml. Then, the resulting solution was filtered through a membrane filter with a pore size of 0.2 μm, and placed in a 10-ml ampule for injection, whereby an injectable solution was obtained.

Example 10: Injectable solution

After 5% by weight of any of the following active ingredients of the invention of this application was dissolved in 5% dextrose in water for injection in accordance with the formulation (% by weight) shown in the following Table 4, the resulting solution was filtered through a membrane filter with a pore size of 0.2 μm and pyrogen was removed by a standard method: Then, an injectable solution of the preventive/therapeutic composition for a free radical disease of the present invention was produced.

Table 4

| Blending example No. | % by weight | Component Type and blending concentration of active ingredient | |
|---|---|---|---|
| 1 | 97% of 5% dextrose in water for injection | Formulation No. 1 in Table 1 | 3% by weight |
| 2 | 97% of 5% dextrose in water for injection | Formulation No. 2 in Table 1 | 3% by weight |
| 3 | 97% of 5% dextrose in water for injection | Formulation No. 3 in Table 1 | 3% by weight |
| 4 | 97% of 5% dextrose in water for injection | Formulation No. 5 in Table 1 | 3% by weight |
| 5 | 97% of 5% dextrose in water for injection | Formulation No. 6 in Table 1 | 3% by weight |
| 6 | 97% of 5% dextrose in water for injection | Formulation No. 7 in Table 1 | 3% by weight |
| 7 | 97% of 5% dextrose in water for injection | Formulation No. 8 in Table 1 | 3% by weight |
| 8 | 97% of 5% dextrose in water for injection | Formulation No. 9 in Table 1 | 3% by weight |
| 9 | 97% of 5% dextrose in water for injection | Formulation No. 10 in Table 1 | 3% by weight |

(continued)

| Blending example No. | % by weight | Component Type and blending concentration of active ingredient | |
|---|---|---|---|
| 10 | 97% of 5% dextrose in water for injection | Formulation No. 11 in Table 1 | 3% by weight |
| 11 | 97% of 5% dextrose in water for injection | Formulation No. 12 in Table 1 | 3% by weight |
| 12 | 97% of 5% dextrose in water for injection | Formulation No. 13 in Table 1 | 3% by weight |

Example 11: Injectable solution for infusion

5 g of each compound (formulation Nos. 1 to 10) shown in the above Table 1 subjected to a sterilization treatment was dissolved in 500 ml of 5% dextrose in water for infusion and the resulting solution was filtered through a membrane filter with a pore size of 0.2 μm, whereby an injectable solution for infusion was obtained.

Example 12: Capsule

20 mg of each compound (formulation Nos. 1 to 10) shown in the above Table 1, 70 mg of cornstarch and 5 mg of magnesium monostearate were mixed and the resulting mixture was packed in a gelatin capsule, whereby' a capsule was obtained.

Example 13: Powdered drug

1 g of each compound (formulation Nos. 1 to 10) shown in the above Table 1 and 10 g of crystalline lactose were mixed, whereby a powdered drug was obtained.

Example 14: Film-coated tablet

20 mg of each compound (formulation Nos. 1 to 10) shown in the above Table 1, 20 mg of potato starch, 30 mg of crystalline cellulose, 20 mg of lactose, 15 mg of calcium hydrogen monoester anhydride, 5 mg of sucrose fatty acid ester and 3 mg of magnesium aluminometasilicate were tableted, and then, a film-coated tablet was obtained with 10 mg of hydroxypropylmethyl cellulose.

Example 15: Oral syrup

Each compound (formulation Nos. 1 to 10) shown in the above Table 1 was suspended in an oral syrup liquid, whereby an oral syrup was obtained.

Example 16: Ointment

In accordance with the formulation shown in the following Table 5 and by a standard method, a preparation for external use (ointment) of the preventive/therapeutic composition for a free radical disease of the present invention was produced.

Table 5

| | |
|---|---|
| Squalane | 10.0 |
| Stearate monoester | 10.0 |
| Propylene glycol monostearate | 3.0 |
| polyoxyethylene cetylether | 1.0 |
| Propylene glycol | 15.0 |
| Paraben | 0.2 |
| PVP/fullerene (molecular weight of PVP: 60,000) | 5.0 |
| Purified water | balance |

Example 17: Liquid

In accordance with the formulation shown in the following Table 6 and by a standard method, a liquid of the preventive/therapeutic composition for a free radical disease of the invention of this application was produced.

Table 6

| | |
|---|---|
| PVP/fullerene (molecular weight of PVP: 60,000) | 5.0 |
| methyl p-hydroxybenzoate | 0.1 |
| Sodium citrate | 0.5 |
| Citric acid | 0.05 |
| Purified water | balance |

B-2: Evaluation

Test example 1: Stability test

A difference in the stabilities of PVP/fullerene (molecular weight of PVP: 60,000), a fullerene monoester, and salts of a fullerene monoester in an aqueous solution (a storage test at room temperature for 10 days) is shown in Table 7. It is found that the fullerenes are stable regardless of the type thereof, however, ascorbic acid is liable to be hydrolyzed over time even in purified water.

Table 7

| Type of fullerene | Residual ratio after 10 days (%) |
|---|---|
| Fullerene monoester | 99 |
| PVP/fullerene | 100 |
| K salt of fullerene monoester | 98 |
| Na salt of fullerene monoester | 98 |
| Ascorbic acid | 0 |

Incidentally, the stability was evaluated by determining the residual ratio of the above substance using the HPLC method by leaving a 0.1% aqueous solution of the above substance (in purified water) at room temperature for 10 days.

Test example 2: Test for intracellular absorption (intracellular transport)

Bovine aortic endothelial cells BAE2 were inoculated at a cell density of 8 x $10^4$/cm$^2$, and after 18 hours, each of a fullerene, ascorbic acid and the fullerenes of the invention of this application of the formulation Nos. 1 to 13 shown in the above Table 1 was administered at a concentration of 50 $\mu$M which falls within the range of the level of the respective substances in the normal human blood. After 22 hours, the amounts of fullerenes and ascorbic acid present in the cells were determined by coulometry and HPLC (high-performance liquid chromatography) with ECD (electrochemical detection). The water content in the cells was measured by gas chromatography using 14C-labeled polyethylene glycol and found to be 0.598 pL/cell. As a result, for example, when PVP/fullerene (molecular weight of PVP: 60,000) and ascorbic acid were administered, the concentrations of fullerene in the cells were 565 $\mu$M and 60 $\mu$M, respectively, and it was confirmed that the fullerene was concentrated in the cells at a concentration 11.3 times and 1.2 times higher than that outside the cells, respectively, and accumulated therein. In the case of the other fullerenes, almost the same results were obtained. In this way, it was shown that the intracellular transport of a fullerene is dramatically superior to that of ascorbic acid.

Test example 3: Test for cell migration (cell haptotaxis)

A Boyden double chamber was used, in which a porous film with 8 $\mu$m pores was placed, the upper surface of the porous film was coated with Matrigel as a reconstituted basement membrane, the lower surface of the porous film was coated with fibronectin as an extracellular matrix. In the upper compartment of the chamber, 2 x $10^5$ cells of a human fibrosarcoma cell line HT1080 were inoculated, and after 3.5 hours, Diff Quick staining was carried out and the

number of invasive cancer cells invading the lower compartment was counted using a CCD camera/ multibioscanner, and the cancer metastasis inhibitory action of the fullerene of the invention of this application was confirmed. To the cancer cells, ascorbic acid or any of the fullerenes of the invention of this application of the formulation Nos. 1 to 13 shown in the above Table 1 was administered for 18 hours in advance. As a result, the number of invasive cancer cells in the case where no agent was administered was 12400, however, as the agent was administered, the number of the cells significantly decreased in any case. For example, in the case of PVP/fullerene (molecular weight of PVP: 60,000), by the administration thereof at 300 μM and 400 μM, the number of cells decreased to 8960 and 356, respectively. Further, the concentration of ascorbic acid present in the cells was determined by coulometry and HPLC with ECD. As a result, in the case where ascorbic acid and any of the fullerenes of the invention of this application of the formulation Nos. 1 to 13 shown in the above Table 1 were administered at 300 μM, the concentration was 103, and 2700 to 2800 μM, respectively. From these results, it was confirmed that cancer cell metastasis is most inhibited in the administration group of the fullerene of the invention of this application, and further, it was suggested that this effect was obtained because the fullerene of the invention of this application increases the intracellular concentration of fullerene most.

Test example 4: Effect test I

Rat spontaneous breast cancer cells (SST-2) ($1 \times 10^5$ cells) were subcutaneously transplanted into the back of a rat (SHR rat) suffering from spontaneous hypertension (each group consisting of 5 rats). After 35 days, the rat was sacrificed, and the lung weight and the number of colonies formed were measured, and the extent of lung metastasis of the SST-2 was observed. In an administration group of the compound of the invention of this application of the formulation No. 1 shown in the above Table 1, the compound was orally administered or subcutaneously injected at a dose of 50 or 100 mg/rat over the period from day 7 before the transplantation to day 34 after the transplantation. Then, the subcutaneous tumor weight, the lung weight, and the number of metastatic colonies formed were compared with those of a control group to which no treatment was provided. The results are shown in Table 8.

Table 8

| Dose of the compound of the present invention (mg/rat) | | Subcutaneous tumor weight (g) | Lung weight (g) | Average number of metastatic colonies formed |
|---|---|---|---|---|
| | 0 | 41.3 | 4.1 | 94.6 |
| Oral administration | 50 | 45.6 | 1.8 | 32.1 |
| Oral administration | 100 | 43.2 | 1.4 | 20.3 |
| Subcutaneous injection | 50 | 44.1 | 1.5 | 21.8 |
| Subcutaneous injection | 100 | 43.7 | 1.4 | 15.3 |

From the results, an inhibitory effect on lung metastasis was confirmed in the administration group of the compound of the present invention.

Test example 5: Effect test II

Rat spontaneous breast cancer cells (SST-2) ($1\times10^5$ cells) were subcutaneously transplanted into the back of a rat (SHR rat) suffering from spontaneous hypertension (each group consisting of 5 rats). After 35 days, the rat was sacrificed, and the lung weight and the number of colonies formed were measured, and the extent of lung metastasis of the SST-2 was observed. A test was carried out for a group in which the compound of the invention of this application of the formulation No. 1 shown in the above Table 1 was administered alone; and groups in which the compound of the invention of this application of the formulation No. 1 shown in the above Table 1 was administered in combination with each of the known antitumor agents shown in Table 9. The compound of the invention of this application was orally administered at a dose of 50 mg/day/rat, and each of the known antitumor agents was orally administered at a minimum dose by reference to the concentration for a single use case described in the publication, (Iyakuhin Yoran (Medical Product Handbook), 4th Edition, pp. 1474-1509, Yakugyo Jiho-Sha (1989)) over the period from day 7 before the transplantation to day 34 after the transplantation. Then, the subcutaneous tumor weight, the lung weight, and the number of metastatic colonies formed were compared with those of the administration group of the compound of the invention of this application. Those in which a cancer metastasis inhibitory effect was observed compared with the administration group of the compound of the invention of this application was indicated by "O", those in which such an

effect was not observed was indicated by "x". The results are shown in Table 9.

Table 9

| Known anti-malignant tumor agent used in combination with the compound of the present invention | Result |
|---|---|
| Nitromin (R) | ○ |
| Cyclophosphamide | ○ |
| Merphalan | ○ |
| Thiotepa | ○ |
| Carboquone | ○ |
| Protecton (R) | ○ |
| Busulfan | ○ |
| Nimustine hydrochloride | ○ |
| Mitobronitol | ○ |
| Ifosfamide | ○ |
| Mercaptopurine | ○ |
| Thioinosine | ○ |
| Cytarabine | ○ |
| Dacarbazine | ○ |
| Fluorouracil | ○ |
| Tegaful | ○ |
| Ancitabine hydrochloride | ○ |
| Methotrexate | ○ |
| Carmofur | ○ |
| UFT (R) | ○ |
| Enocitabine | ○ |
| Vinblastine sulfate | ○ |
| Vincristine sulfate | ○ |
| Vindesine sulfate | ○ |
| Actinomycin (D) | ○ |
| Mitomycin C | ○ |
| Chromomycin A3 | ○ |
| Bleomycin hydrochloride | ○ |
| Bleomycin sulfate | ○ |
| Daunorubicin hydrochloride | ○ |
| Doxorubicin hydrochloride . | ○ |
| Neocarzinostatin | ○ |
| Peplomycin sulfate | ○ |
| Aclarubicin hydrochloride | ○ |
| Mepitiostane | ○ |
| Epitiostanol | ○ |
| Tamoxifen citrate | ○ |
| Honvan | ○ |
| Picibanil (R) | ○ |
| Krestin | ○ |
| Lentinan | ○ |
| L-Asparaginase | ○ |
| Aceglatone | ○ |
| Procarbazine hydrochloride | ○ |
| Floxuridine | ○ |
| MDS KOWA 3000 (R) | ○ |
| Cisplatin | ○ |
| Estracyt (R) | ○ |

(continued)

| Known anti-malignant tumor agent used in combination with the compound of the present invention | Result |
|---|---|
| Sizofiran | ○ |
| Protamine | ○ |
| Angiostatic steroid containing heparin | ○ |
| Peptide-glycan complex | ○ |
| GDPGYIGSR-NH2 | ○ |
| Antitumor polypeptide | ○ |
| Platelet factor-4 | ○ |

From these results, it was confirmed that in the group in which the compound of the invention of this application and a known antitumor agent were used in combination, a higher inhibitory effect on lung metastasis was exhibited compared with the case in which the compound of the invention of this application was administered alone.

Test example 6: Evaluation of inhibitory effect on skin cancer metastasis in mouse

The compound of the formulation No. 1 shown in the above Table 1 was administered to a mouse under the following conditions, and an inhibitory effect on skin cancer metastasis in the mouse was evaluated.

(Mouse): C57BL/6, 8 weeks old, female
(Cell line): B16BL6 (1 x $10^5$ cells/0.2 ml)
(Administration method): Injection via the tail vein, 5 consecutive administration
(Rearing period): 14 days
The results are shown in Fig. 1. It was confirmed that a PVP/fullerene complex inhibits skin cancer metastasis.

Test example 7: Cancer invasion test

By using the compound of the formulation No. 1 shown in the above Table 1, that is, PVP/fullerene (molecular weight of PVP: 60,000) and using an invasive cell line, B16BL6 (15 scells/0.2 ml) in the same manner as in Test example 3, a cancer invasion test was carried out. In the test, a "simultaneous treatment" in which PVP/fullerene was administered immediately before the cancer cells were allowed to invade into a reconstituted basement membrane, and a "hr pretreatment" in which PVP/fullerene was administered at 1 hour before the cancer cells were allowed to invade into a reconstituted basement membrane and culture was carried out for 1 hour while mildly stirring thereby to prevent cell adhesion, then, the cancer cells were allowed to invade were carried out.

Incidentally, 40 μl of Matrigel (0.12 mg/ml) was used. The results were compared with those of the case of nonadministration and the case of administration of only PVP (molecular weight of PVP: 60,000). The results are shown in Fig. 2.

As shown in Fig. 2, it was confirmed that PVP/ fullerene significantly suppresses cancer cell metastasis.

Industrial Applicability

As described above, according to the preventive/ therapeutic composition for a free radical disease of the present invention, a preventive/therapeutic agent for a wide variety of free radical diseases, that is, an agent having an effective activity for adult diseases, cancer and cancer metastasis inhibition, infectious diseases and in particular, various ischemic diseases is provided because the composition is stable without losing its activity in a pharmaceutical preparation and in the living body, has high safety and less side effects, and further has an excellent free radical scavenging activity compared with an agent for a radical disease containing a conventional antioxidant as an active ingredient, which is easily oxidized, and in which it is difficult to keep its quality while or after it is formulated into a preparation. The composition of the present invention is widely useful as a preventive or therapeutic agent for a free radical disease which has been recognized as a social problem.

## Claims

**1.** A preventive/therapeutic composition for a free radical disease, **characterized by** comprising as an active ingredient, at least one kind of a fullerene, a fullerene derivative, and a complex of a fullerene or a fullerene derivative modified

with or clathrated in an organic compound.

2. The preventive/therapeutic composition for a free radical disease according to claim 1, wherein the fullerene derivative is a fullerene linked to at least one kind of an oxygen-containing group, a nitrogen-containing group and a hydrocarbon group which may have a substituent.

3. The preventive/therapeutic composition for a free radical disease according to claim 1, wherein the fullerene is at least one kind selected from a fullerene polymer, a carbon nanotube, a derivative thereof, and a salt thereof.

4. The preventive/therapeutic composition for a free radical disease according to claim 1, wherein the fullerene is at least one kind selected from a cleaved form of a fullerene, a split form of a fullerene, a derivative thereof and a salt thereof.

5. The preventive/therapeutic composition for a free radical disease according to claim 3 or 4, wherein the salt of a fullerene is at least one kind of salts of sodium, potassium, magnesium, calcium and aluminum.

6. The preventive/therapeutic composition for a free radical disease according to any one of claims 1 to 5, wherein the fullerene is a complex of at least one kind of a fullerene and a fullerene derivative with at least one kind of an organic oligomer, an organic polymer, cyclodextrin, crown ether and an analogous compound thereof.

7. The preventive/therapeutic composition for a free radical disease according to claim 6, which is a complex of at least one kind of a fullerene and a fullerene derivative with polyvinylpyrrolidone (PVP).

8. The preventive/therapeutic composition for a free radical disease according to any one of claims 1 to 5, wherein the fullerene is represented by the following formula:

**R1m-F-R2m**

(wherein F denotes a fullerene represented by Cn (C denotes a carbon atom and n denotes an integer of 32 or more), a carbon nanotube, a polymer thereof, a cleaved form thereof, a split form thereof or a mixture thereof; R1m denotes m-number of substituents linked to Cn, the substituents are independently the same or different and represent a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; R2m denotes m-number of atoms linked to Cn, the atoms are independently the same or different or one atom is linked to 2 or more different carbons; and m denotes 0 or an integer of 1 or more).

9. The preventive/therapeutic composition for a free radical disease according to claim 4 or 8, wherein the cleaved form or split form of a fullerene is a fullerene represented by any of the following formulae or a single substance or a complex of a molecule having the structure therein:

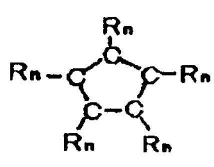

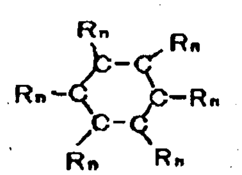

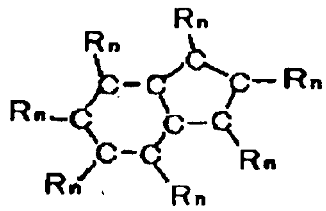

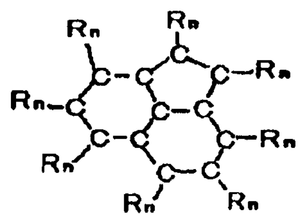

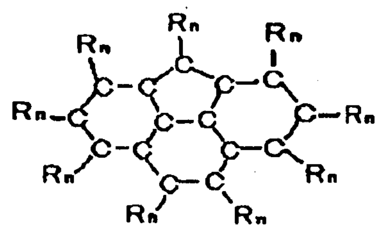

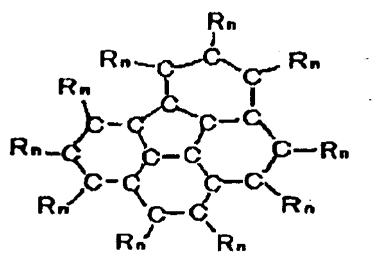

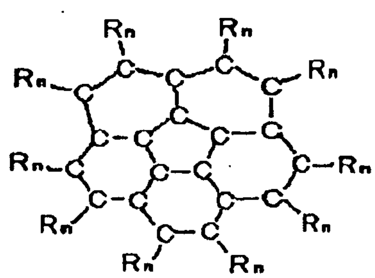

(wherein C denotes a partial structure of a fullerene represented by a carbon atom and 2 or more carbon atoms may be linked via a covalent bond (in the case where a carbon which is not covalently linked is present, even if there is no denotation by Rn in the formula, the carbon is linked to Rn); Rn denotes n-number of substituents linked to C, the substituents are independently the same or different and represent a hydrogen atom, a carbon atom, an oxygen atom, a nitrogen atom, a phosphorous atom, a hydroxyl group, an ester group of the hydroxyl group with an inorganic acid or an organic acid, a glycoside group of the hydroxyl group with a saccharide, a ketal group of the hydroxyl group with a ketone or an acetal group of the hydroxyl group with an aldehyde; and n denotes 0 or

an integer of 1 or more).

**10.** The preventive/therapeutic composition for a free radical disease according to any one of claims 1 to 9, further comprising at least one kind of antitumor agent.

**11.** The preventive/therapeutic composition for a free radical disease according to claim 10, wherein the antitumor agent is at least one kind of nitromin (R), cyclophosphamide, merphalan, thiotepa, carboquone, Protecton (R), busulfan, nimustine hydrochloride, mitobronitol, ifosfamide, mercaptopurine, thioinosine, cytarabine, dacarbazine, fluorouracil, tegaful, ancitabine hydrochloride, methotrexate, carmofur, UFT (R), enocitabine, vinblastine sulfate, vincristine sulfate, vindesine sulfate, actinomycin (D), mitomycin C, chromomycin A3, bleomycin hydrochloride, bleomycin sulfate, daunorubicin hydrochloride, doxorubicin hydrochloride, neocarzinostatin, peplomycin sulfate, aclarubicin hydrochloride, mepitiostane, epitiostanol, tamoxifen citrate, Honvan, Picibanil (R), krestin, lentinan, L-asparaginase, aceglatone, procarbazine hydrochloride, floxuridine, MDS KOWA 3000 (R), cisplatin, Estracyt (R), Sizofiran, protamine, an angiostatic steroid in the presence of heparin, an antitumor polysaccharide, a laminin peptide, an antitumor polypeptide containing an Arg-Gly-Asp (RGD) sequence and an antitumor platelet factor.

**12.** The preventive/therapeutic composition for a free radical disease according to any one of claims 1 to 11, wherein the free radical disease is myocardial infarction, an ischemic heart disease, heart failure, angina pectoris, cardiac arrhythmia, arteriosclerosis, disturbance of lipid metabolism in the liver, hyperlipemia, essential hypertension, hypertension, arteriosclerosis, coronary arteriosclerosis, thrombosis, arteriosclerosis obliterans, vascular disorder, peripheral vascular disorder, cholestasis, hypercholesterolemia, pancreatic damage, organ failure, acute or chronic hepatitis, gastric ulcer, duodenal ulcer, colitis ulcerosa, digestive system damage, cholecystopathy, diabetes, arthritis therapeutic agent, rheumatoid, liver failure, liver damage, ischemic liver damage, disturbance of lipid metabolism in the liver, gallbladder damage, organ transplantation damage, diabetes, toxicosis, organ transplantation damage, cancer, ischemic reperfusion damage, tissue aging, skin pigmentation, skin wrinkle, alveolar pyorrhea, skin seborrhea, skin tanning, skin acne, burn injury, obesity, hair loss, mental disorder, dementia, Parkinson's disease or an infectious disease.

**13.** The preventive/therapeutic composition for a free radical disease according to claim 12, wherein the cancer is a tumor, a benign tumor or a malignant tumor.

**14.** The preventive/therapeutic composition for a free radical disease according to claim 12, wherein the cancer is malignant melanoma, malignant lymphoma, gastrointestinal cancer, lung cancer, esophageal cancer, stomach cancer, large bowel cancer, rectal cancer, colon cancer, ureteral tumor, gallbladder cancer, bile duct cancer, biliary tract cancer, breast cancer, liver cancer, pancreatic cancer, testicle tumor, maxillary cancer, tongue cancer, lip cancer, oral cavity cancer, pharyngeal cancer, larynx cancer, ovary cancer, uterine cancer, prostate cancer, thyroid cancer, brain tumor, Kaposi's sarcoma, hemangioma, leukemia, polycythemia vera, neuroblastoma, retinoblastoma, myeloma, bladder tumor, sarcoma, osteogenic sarcoma or myosarcoma.

**15.** The preventive/therapeutic composition for a free radical disease according to claim 12, wherein the cancer is skin cancer, basal cell cancer, skin appendage carcinoma, skin metastasis cancer or skin melanoma.

**16.** The preventive/therapeutic composition for a free radical disease according to claim 12, wherein the ischemic reperfusion damage is an ischemic heart disease, ischemic reperfusion myocardial damage, ischemic liver damage, ischemic reperfusion liver damage, ischemic reperfusion renal damage, ischemic reperfusion pancreatic damage, ischemic reperfusion gallbladder damage, ischemic reperfusion cardiovascular damage, ischemic reperfusion gastrointestinal damage, ischemic reperfusion muscle damage, ischemic reperfusion vascular damage, ischemic reperfusion eye damage or ischemic reperfusion skin damage.

**17.** The preventive/therapeutic composition for a free radical disease according to claim 12, wherein the infectious disease is a bacterial infectious disease, a viral infectious disease, a fungal infectious disease or a parasitic infectious disease.

**18.** The preventive/therapeutic composition for a free radical disease according to claim 17, wherein the viral infectious disease is hepatitis, acquired immunodeficiency syndrome (AIDS), common cold or influenza.

Fig. 1

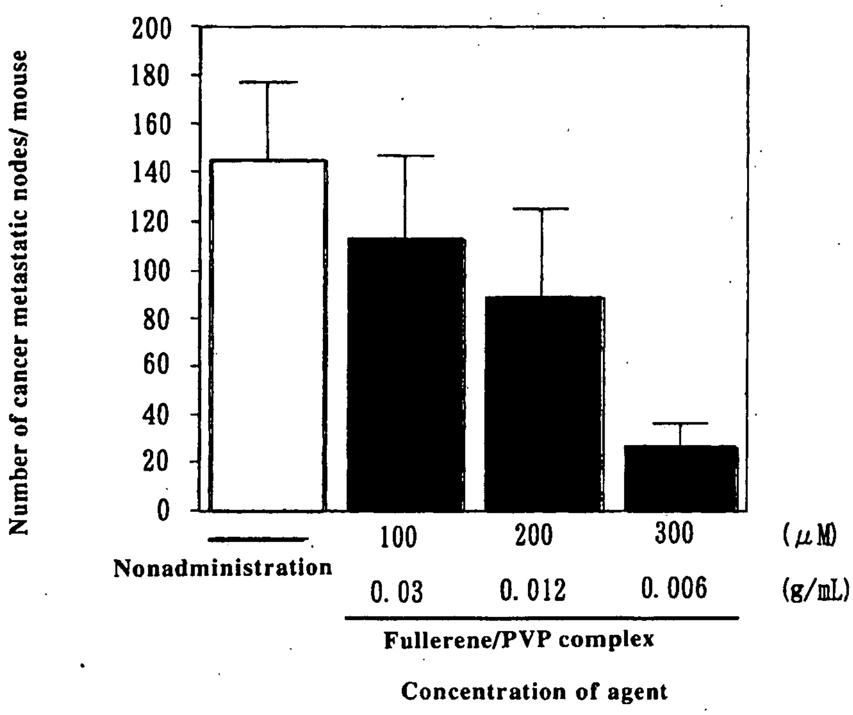
Number of cancer metastatic nodes/ mouse
200
180
160
140
120
100
80
60
40
20
0
Nonadministration
100
200
300
(μM)
0.03
0.012
0.006
(g/mL)
Fullerene/PVP complex
Concentration of agent

Fig. 2

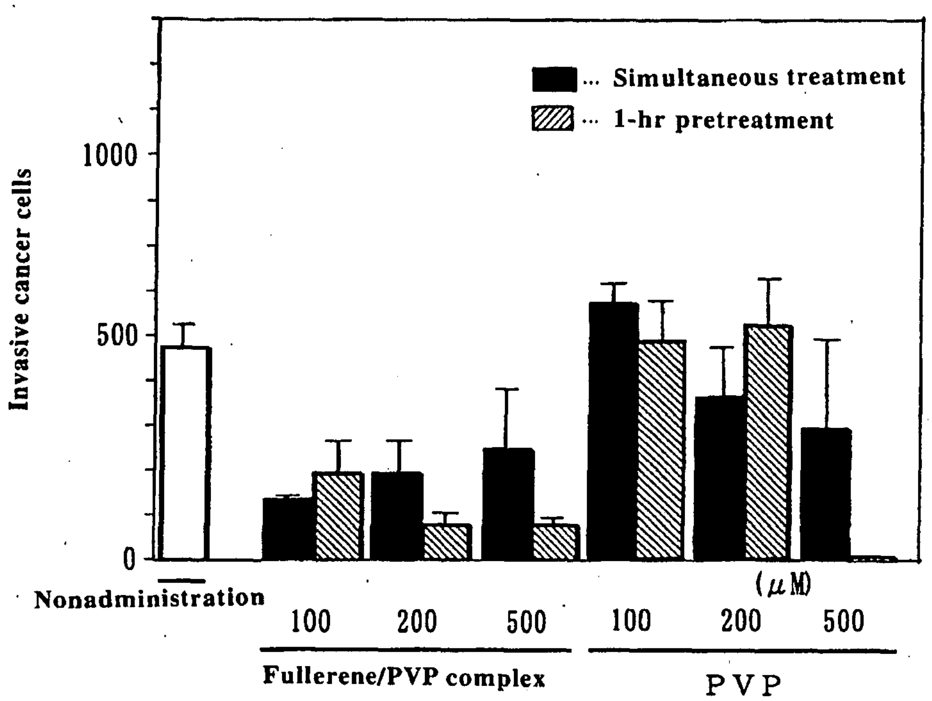
... Simultaneous treatment
... 1-hr pretreatment
Invasive cancer cells
1000
500
0
Nonadministration
100
200
500
100
200
500
(μM)
Fullerene/PVP complex
PVP

## INTERNATIONAL SEARCH REPORT

International application No.
PCT/JP2005/014798

A. CLASSIFICATION OF SUBJECT MATTER
Int.Cl$^7$ A61K31/01, 7/00, 7/48, 31/02, 31/045, 31/395, 33/44, 47/40, A61P1/02, 1/04, 1/14, 1/16, 1/18, 3/04, 3/06, 3/10, 7/02, 9/04, 9/06, 9/08, 9/10, 9/12, 17/00, 17/02, 17/08, 17/10, 17/14, 17/16, 19/02, 25/16,

According to International Patent Classification (IPC) or to both national classification and IPC

B. FIELDS SEARCHED

Minimum documentation searched (classification system followed by classification symbols)
Int.Cl$^7$ A61K31/01, 7/00, 7/48, 31/02, 31/045, 31/395, 33/44, 47/40, A61P1/02, 1/04, 1/14, 1/16, 1/18, 3/04, 3/06, 3/10, 7/02, 9/04, 9/06, 9/08, 9/10, 9/12, 17/00, 17/02, 17/08, 17/10, 17/14, 17/16, 19/02, 25/16,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAOLD(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2004-250690 A (Mitsubishi Corp.),<br>09 September, 2004 (09.09.04)<br>& WO 2004-67678 A1 | 1-9,12-18<br>10-11 |
| X<br><br>A | JP 2004-231595 A (Mitsubishi Chemical Corp.),<br>19 August, 2004 (19.08.04)<br>(Family: none) | 1-2,8-9,<br>12-18<br>3-7,10-11 |
| X<br>A | JP 2004-292443 A (Mitsubishi Chemical Corp.),<br>21 October, 2004 (21.10.04)<br>& WO 2004-80453 A1 | 1-2,9,12-18<br>3-8,10-11 |
| X<br>A | JP 2002-504525 A (Akushiba GMBH),<br>12 February, 2002 (12.02.02)<br>& US 6506928 A & WO 99/43358 A1 | 1-2,9,12-18<br>3-8,10-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 September, 2005 (02.09.05) | 20 September, 2005 (20.09.05) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2005/014798

C (Continuation). DOCUMENTS CONSIDERED TO BE RELEVANT

| Category* | Citation of document, with indication, where appropriate, of the relevant passages | Relevant to claim No. |
|---|---|---|
| X<br>A | BOGDANOVIC, G. et al., Modulating activity of fullerol $C_{60}(OH)_{22}$ on doxorubicin-induced cytotoxicity, Toxicology In Vitoro, 2004.08, Vol.18, No.5, pages 629 to 637 | 1-2,5,8-18<br>3-4,6-7 |

Form PCT/ISA/210 (continuation of second sheet) (January 2004)

**INTERNATIONAL SEARCH REPORT**

International application No.
PCT/JP2005/014798

Continuation of A. CLASSIFICATION OF SUBJECT MATTER
(International Patent Classification (IPC))

Int.Cl[7] 25/18, 29/00, 31/00, 31/16, 31/18, 35/00, 37/02, 39/06

(According to International Patent Classification (IPC) or to both national classification and IPC)

Continuation of B. FIELDS SEARCHED
Minimum documentation searched (International Patent Classification (IPC))

Int.Cl[7] 25/18, 29/00, 31/00, 31/16, 31/18, 35/00, 37/02, 39/06

Minimum documentation searched (classification system followed by classification symbols)

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6192039 A
- JP 9278625 A
- JP 9322767 A
- JP 2004250690 A
- JP 57042616 A
- JP 57193462 A
- JP 59027823 A
- JP 60028963 A
- JP 59163353 A
- JP 59190991 A
- JP 61037766 A
- JP 4273817 A
- JP 61044853 A
- JP 61065869 A
- JP 61282377 A
- JP 63301821 A
- JP 63150276 A
- JP 1143868 A
- JP 1146820 A
- JP 3176487 A
- JP 3236378 A
- JP 4211666 A
- JP 1025726 A
- JP 4330088 A
- JP 54004401 A
- JP 2308799 A
- JP 4312531 A
- JP 6072871 A
- JP 6107693 A
- JP 8291075 A
- JP 10330261 A


### Non-patent literature cited in the description


- Supersonic Carbon Cluster Beams in Atomic and Molecular Clusters. **R. E. SMALLEY.** Physical and Theoretical Chemistry. Elsevier Science, 1990, vol. 68, 1-68
- **R. F. CURL et al.** Fullerenes. *Scientific American,* October 1991, 32-41
- **F. DIEDERICH et al.** The Higher Fullerenes: Isolation and Characterization. *Science,* vol. 252, 548-551
- **R. E. SMALLEY.** Great Balls of Carbon: The Story of Buckminsterfullerene. *The Sciences,* March 1991, vol. 31 (2), 22-28
- **W. KRAETSCHMER et al.** *Nature,* 1990, vol. 347, 354-358
- *Chemical and Engineering News,* October 1990, 22-25
- Kassei Sanso to Byotai. Japan Scientific Societies Press, 1992
- *Kasanka Busshitsu,* 1994
- **INOUE et al.** *Biochemistry,* 1989, vol. 28, 6619-6624
- Kosanka Busshitsu. Japan Scientific Societies Press, 1994
- *Gendai Iryo,* 1993, vol. 25 (10
- **OBAYASHI.** *Proc. Soc. Exp. Biol. Wed.,* 1990, vol. 196 (196), 164-169
- **NARITA, W. J. J.** *Lab. Clin. Med.,* 1987, vol. 110, 153-158
- **SMITH, L. ; L. PHIL.** *Trans. R. Soc. Lond.,* 1985, vol. 311, 647-657
- **INOUE, M.** Inmucosal Immunology. Elsevier, 1990, 527-530
- **NAKAHAMA.** *Kanzo (Liver,* 1991, vol. 32, 1110-1123
- **TAKEKAWA.** *Kanzo (Liver,* 1989, vol. 30, 459-467
- *Shirosugi, Nippon Shokaki Geka Gakkai Zasshi,* 1993, vol. 26, 358
- **ISAJI, S.** *Mie Med. J.,* 1985, vol. 35, 109-123
- **TAOKA.** *Gastroent. JPN.,* 1991, vol. 26, 653-644
- *Iwai, Nippon Shokaki Geka Gakkai Zasshi,* 1990, vol. 87, 1662-1669
- **NAITO, Y.** *Free Red. Res. Comms.,* 1992, vol. 16, 13. 5
- Japanese Pharmacopoeia. Hirokawa Shoten, 1991
- Iyakuhin Yoran. Yakugyo Jiho-Sha, 1989, 1474-1509